# EUROPEAN PATENT SPECIFICATION

(11) **EP 3 021 948 B1**
(45) Date of publication and mention of the grant of the patent: **21.08.2019**
(21) Application number: 14767114.3
(22) Date of filing: 18.07.2014
(51) Int. Cl.: A61Q 1/00, A61Q 1/06, A61Q 5/00, A61Q 15/00, A61Q 19/00, A61K 8/02, A61K 8/06, A61K 8/81, A61K 8/89, A61K 8/92

(54) **OIL/OIL EMULSIONS CONTAINING PARTICLES WITH A BREAKAGE OF CURVATURE, COMPOSITIONS COMPRISING THEM AND USE OF THE PARTICLES FOR STABILIZING O/O EMULSIONS**
ÖL/ÖL-EMULSIONEN MIT PARTIKELN MIT KRÜMMUNGSBRUCH, ZUSAMMENSETZUNGEN DAMIT UND VERWENDUNG DER PARTIKEL ZUR STABILISIERUNG VON O/O-EMULSIONEN
ÉMULSIONS DE PICKERING HUILE/HUILE RENFERMANT DES PARTICULES À RUPTURE DE COURBE, COMPOSITIONS LES COMPRENANT ET UTILISATION DE CES PARTICULES POUR STABILISER DES ÉMULSIONS HUILE/HUILE

(30) Priority: 18.07.2013 FR 1357076
(43) Date of publication of application: 25.05.2016
(73) Proprietor: L'OREAL, 75008 Paris (FR)
(72) Inventor: LEVY, Florence, F-75011 Paris (FR); HENAULT-MEZAIZE, Léonora, 94550 Chevilly-Larue (FR); PEREZ NOWAK, Virginie, F-94240 L'Hay les Roses (FR)
(74) Representative: Nony
(86) International application number: PCT/IB2014/063224
(87) International publication number: WO 2015/008263

(56) References cited:
- EP-A1- 2 298 273
- WO-A1-2013/190704
- WO-A1-2013/190707
- WO-A2-2004/003115
- FR-A1- 2 912 656
- JP-A- 2000 053 530
- US-A1- 2007 189 998
- US-A1- 2007 224 246

## Description

The present invention relates to the field of cosmetic compositions based on oil/oil (O/O) emulsions.

There is still a need for novel architectures leading to stable cosmetic compositions that have the comfort properties required by users.

The authors of the present invention have oriented their research towards O/O emulsions. These emulsions are relatively uncommon, but nevertheless have the advantage of having novel properties.

The main problem posed by this type of emulsion is associated with their stability: O/O emulsions are generally stabilized with gelling agents or even emulsifying surfactants and/or (co)polymers.

This type of emulsion is used in particular in lipcare and/or lip makeup products. For example, patent application WO 2009/150 852 is directed towards an oil-in-oil cosmetic composition comprising a hydrocarbon-based non-volatile oil, a silicone non-volatile oil and a fatty acid ester of dextrin, but the said application does not describe O/O emulsions of Pickering type.

Surprisingly and advantageously, the authors of the present invention have used O/O emulsions of Pickering type stabilized with solid particles. The emulsions according to the invention comprise two different immiscible oily phases, one forming the continuous phase and the other forming the dispersed phase, and particular solid particles for stabilizing the emulsion by positioning themselves at the interface of the dispersed and continuous phases.

Once positioned at the interface, the solid particles "block" the dispersed phase, which leads to stabilization of the emulsion. The O/O emulsion thus formed is stable for several weeks.

According to the invention, the solid particles are used as emulsion stabilizers.

The emulsion according to the invention also makes it possible to dispense with the use, as stabilizers, of compounds of surfactant type, especially synthetic surfactants, and/or of gelling agents, since some of these agents may present toxicity risks to the environment depending on the amounts used.

In addition, in the context of the invention, the oils used for forming the two phases may be judiciously chosen as a function of the intended use of the final product and of the desired properties.

Thus, a first subject of the invention is directed towards an oil/oil (O/O) emulsion stabilized with solid particles comprising at least:
- a first oily phase comprising at least a first non-volatile oil chosen from silicone oils, hydrocarbon-based oils and fluoro oils, preferably chosen from silicone oils and fluoro oils,
- a second oily phase comprising at least a second non-volatile or volatile oil, preferably a non-volatile oil, which is immiscible with the first oil(s), at 25°C,
- solid microparticles having at least one curved part and at least one breakage of curvature of the said curved part, in which the solid microparticles comprise at least one concave part and at least one convex part.

The invention is also directed towards a cosmetic composition comprising, in a physiologically acceptable medium, at least one O/O emulsion according to the invention.

### Definitions

For the purposes of the present invention, the term "microparticles" is intended to denote a particle whose largest dimension ranges advantageously from 0.1 to 100 µm, preferably from 0.1 to 50 µm and more preferably from 0.5 to 20 µm.

The term "room temperature" is intended to denote a temperature of about 25°C. It is set at atmospheric pressure (i.e. a pressure of 1.013 × 10⁵ Pa).

The compositions according to the invention comprise a physiologically acceptable medium, i.e. a non-toxic medium that can be applied to human skin, and which is of pleasant appearance, odour and feel.

According to a preferred embodiment, the composition according to the invention is a solid composition.

According to another preferred embodiment, the composition according to the invention is a liquid composition.

In particular, it will be a cosmetic composition for caring for and/or making up the skin, and more particularly facial skin, or alternatively a composition for treating keratin fibres.

The term "skin" is intended to denote all of the skin of the body, including the lips, and preferably the skin of the face, the neck and the neckline, and also the lips.

The term "keratin fibres" more particularly means the hair.

More specifically, the composition according to the invention will be a composition chosen from make-up composition, in particular lip makeup products, antisun products, deodorants, care products and fragrances.

In a known manner, the cosmetic composition of the invention may also contain adjuvants that are common in cosmetics, such as lipophilic gelling agents, preserving agents, advantageously saturated C₂-C₅ monoalcohols, fragrances, fillers, UV-screening agents, which are especially lipophilic, bactericides, odour absorbers, dyestuffs, plant extracts, antioxidants and nonionic, anionic, cationic or amphoteric surfactants.

The amounts of these various adjuvants are those conventionally used in the field under consideration, and are for example from 0.01% to 20% of the total weight of the composition. Depending on their nature, these adjuvants may be introduced into the first oily phase and/or into the second oily phase.

According to another subject, the invention is directed towards the use of solid microparticles having at least one curved part and at least one breakage of curvature of the said curved part, in which the solid microparticles comprise at least one concave part and at least one convex part, for stabilizing an oil/oil (O/O) emulsion comprising at least a first oily phase comprising at least one first oil chosen from silicone oils, hydrocarbon-based oils and fluoro oils, and at least a second oily phase comprising at least a second oil that is immiscible with the first oil(s) at 25°C.

### Solid particles

The solid microparticles that may be used for stabilizing the O/O emulsion according to the invention have a particular form, they thus have at least one curved part and at least one breakage of curvature of the said curved part, and comprise at least one concave part and at least one convex part.

Thus, the solid microparticles that may be used in the present invention comprise several curvatures.

The term "several curvatures" means curvatures of different radius.

For the purposes of the present invention, the term "radius of curvature" does not cover the "infinity" value "∞", thus, the microparticles used according to the invention are not in the form of platelets or leaflets.

More particularly, the microparticles in accordance with the invention have a form chosen from forms of "bowl", "golfball" and "polytope" type.

The O/O emulsion according to the invention may further comprise solid microparticles comprising only one curvature.

For the purposes of the invention, the term "only one curvature" means that when the microparticles comprise several curves, these curves have curvatures of the same radius.

They are chosen especially from hemispherical, fusiform microparticles, for example of "rugby ball" type.

The solid microparticles that may be used in the present invention may be mineral or organic.

In general, the microparticles that may be used in the present invention are such that their largest dimension ranges from 0.1 to 100 µm, preferably from 0.1 to 50 µm and more preferably from 0.5 to 20 µm.

Advantageously, the microparticles that may be used in the present invention have a density ranging from 0.5 to 2.8 and preferably from 0.8 to 1.5.

The microparticles may be polar or apolar, and are preferably apolar.

The microparticles according to the invention are generally obtained by radical polymerization or by polycondensation.

The term "radical polymerization" means polymerization of at least one ethylenic monomer.

In this case, preferably, the microparticles according to the invention contain, or even consist of, a polymer chosen from polyacrylates, polymethyl methacrylate (PMMA) and polystyrenes.

The term "polycondensation" means polymerization between two monomers with elimination of a small molecule.

In this case, preferably, the microparticles according to the invention contain, or even consist of, a polymer chosen from polysilicones, polyurethanes and polyesters.

### Bowls

According to a first variant of the invention the microparticles more particularly have a hollow hemispherical shape, i.e. a "bowl" shape.

The "bowl"-shaped microparticles may comprise or consist of PMMA, and/or of a silicone material, and preferably comprise or consist of a silicone material.

The latter preferred embodiment is described in detail below under the name "concave particles of silicone material".

The "bowl"-shaped microparticles made of crosslinked polymethyl methacrylate (PMMA) are especially the product Micropearl® M310 sold by the company SEPPIC.

### Concave particles of silicone material

The concave particles present in the composition according to the invention are silicone particles, in particular hollow sphere portions consisting of a silicone material.

The said particles preferably have a mean diameter ranging from 0.1 µm to 20 µm and preferentially from 0.5 to 15 µm. The term "mean diameter" means the largest dimension of the particle.

The hollow sphere portions used in the composition according to the invention have the form of truncated hollow spheres, having only one orifice communicating with their central cavity, and having a horseshoe-shaped or bowl-shaped cross section.

The silicone material is a crosslinked polysiloxane of three-dimensional structure; it preferably comprises, or even consists of, units of formula (I) SiO₂ and of formula (II) R¹SiO_{1.5}, in which R¹ denotes an organic group bearing a carbon atom directly linked to the silicon atom.

Advantageously, the solid microparticles are in the form of bowls and comprise, or even consist of, units of formula (I) SiO₂ and of formula (II) R¹SiO_{1.5}, in which R¹ denotes an organic group bearing a carbon atom directly linked to the silicon atom.

The organic group R¹ may be a reactive organic group; R¹ may more particularly be an epoxy group, a (meth)acryloxy group, an alkenyl group, a mercaptoalkyl, aminoalkyl or haloalkyl group, a glyceroxy group, a ureido group, a cyano group and, preferably, an epoxy group, a (meth)acryloxy group, an alkenyl group or a mercaptoalkyl or aminoalkyl group. These groups generally contain from 2 to 6 carbon atoms and especially from 2 to 4 carbon atoms.

The organic group R¹ may also be an unreactive organic group; R¹ may then more particularly be a C₁-C₄ alkyl group, especially a methyl, ethyl, propyl or butyl group, or a phenyl group, and preferably a methyl group.

Epoxy groups that may be mentioned include a 2-glycidoxyethyl group, a 3-glycidoxypropyl group and a 2-(3,4-epoxycyclohexyl)propyl group.

(Meth)acryloxy groups that may be mentioned include a 3-methacryloxypropyl group and a 3-acryloxypropyl group.

Alkenyl groups that may be mentioned include vinyl, allyl and isopropenyl groups.

Mercaptoalkyl groups that may be mentioned include mercaptopropyl and mercaptoethyl groups.

Aminoalkyl groups that may be mentioned include a 3-(2-aminoethyl)aminopropyl group, a 3-aminopropyl group and an N,N-dimethylaminopropyl group.

Haloalkyl groups that may be mentioned include a 3-chloropropyl group and a trifluoropropyl group.

Glyceroxy groups that may be mentioned include a 3-glyceroxypropyl group and a 2-glyceroxyethyl group.

A ureido group that may be mentioned is a 2-ureidoethyl group.

Cyano groups that may be mentioned include cyanopropyl and cyanoethyl groups.

Preferably, in the unit of formula (II), R¹ denotes a methyl group.

Advantageously, the silicone material comprises the units (I) and (II) in a unit (I)/unit (II) mole ratio ranging from 30/70 to 50/50 and preferably ranging from 35/65 to 45/55.

The silicone material particles may especially be obtained according to a process comprising:
(a) the introduction into an aqueous medium, in the presence of at least one hydrolysis catalyst, and optionally of at least one surfactant, of a compound (III) of formula SiX₄ and of a compound (IV) of formula RSiY₃, in which X and Y denote, independently of each other, a C₁-C₄ alkoxy group, an alkoxyethoxy group containing a C₁-C₄ alkoxy group, a C₂-C₄ acyloxy group, an N,N-dialkylamino group containing C₁-C₄ alkyl groups, a hydroxyl group, a halogen atom or a hydrogen atom, and R denotes an organic group comprising a carbon atom directly bonded to the silicon atom; and
(b) the placing in contact of the mixture resulting from step (a) with an aqueous solution containing at least one polymerization catalyst and optionally at least one surfactant, at a temperature of between 30 and 85°C, for at least two hours.

Step (a) corresponds to a hydrolysis reaction and step (b) corresponds to a condensation reaction.

In step (a), the mole ratio of compound (III) to compound (IV) usually ranges from 30/70 to 50/50, advantageously from 35/65 to 45/55 and is preferentially 40/60. The weight ratio of water to the total of compounds (III) and (IV) preferably ranges from 10/90 to 70/30. The order of introduction of compounds (III) and (IV) generally depends on their rate of hydrolysis. The temperature of the hydrolysis reaction generally ranges from 0 to 40°C and usually does not exceed 30°C to avoid premature condensation of the compounds.

For the groups X and Y of compounds (III) and (IV):
C₁-C₄ alkoxy groups that may be mentioned include methoxy and ethoxy groups;
As alkoxyethoxy groups containing a C₁-C₄ alkoxy group, mention may be made of methoxyethoxy and butoxyethoxy groups;
C₂-C₄ alkoxy groups that may be mentioned include acetoxy and propioxy groups;
As N,N-dialkylamino groups containing C₁-C₄ alkyl groups, mention may be made of dimethylamino and diethylamino groups;
Halogen atoms that may be mentioned include chlorine and bromine atoms.

Compounds of formula (III) that may be mentioned include tetramethoxysilane, tetraethoxysilane, tetrabutoxysilane, trimethoxyethoxysilane, tributoxyethoxysilane, tetraacetoxysilane, tetrapropioxysilane, tetraacetoxysilane, tetra(dimethylamino)silane, tetra(diethylamino)silane, silane tetraol, chlorosilane triol, dichlorodisilanol, tetrachlorosilane and chlorotrihydrogenosilane. Preferably, the compound of formula (III) is chosen from tetramethoxysilane, tetraethoxysilane and tetrabutoxysilane, and mixtures thereof.

The compound of formula (III) leads, after the polymerization reaction, to the formation of the units of formula (I).

The compound of formula (IV) leads, after the polymerization reaction, to the formation of the units of formula (II).

The group R in the compound of formula (IV) has the meaning as described for the group R¹ for the compound of formula (II).

As examples of compounds of formula (IV) comprising an unreactive organic group R, mention may be made of methyltrimethoxysilane, ethyltriethoxysilane, propyltributoxysilane, butyltributoxysilane, phenyltrimethoxyethoxysilane, methyltributoxyethoxysilane, methyltriacetoxysilane, methyltripropioxysilane, methyltriacetoxysilane, methyltri(dimethylamino)silane, methyltri(diethylamino)silane, methylsilanetriol, methylchlorodisilanol, methyltrichlorosilane and methyltrihydrogenosilane.

As examples of compounds of formula (IV) comprising a reactive organic group R, mention may be made of:
silanes containing an epoxy group, for instance 3-glycidoxypropyltrimethoxysilane, 3-glycidoxypropyltriethoxysilane, 2-(3,4-epoxycyclohexyl)ethyltrimethoxysilane, 3-glycidoxypropylmethyldimethoxysilane, 3-glycidoxypropylmethyldimethoxysilane, 2-glycidoxyethylmethyldimethoxysilane, 3-glycidoxypropyldimethylmethoxysilane and 2-glycidoxyethyldimethylmethoxysilane;
silanes containing a (meth)acryloxy group, for instance 3-methacryloxypropyltrimethoxysilane and 3-acryloxypropyltrimethoxysilane;
silanes containing an alkenyl group, for instance vinyltrimethoxysilane, allyltrimethoxysilane and isopropenyltrimethoxysilane;
silanes containing a mercapto group, for instance mercaptopropyltrimethoxysilane and mercaptoethyltrimethoxysilane;
silanes containing an aminoalkyl group, for instance 3-aminopropyltrimethoxysilane, 3-(2-aminoethyl)aminopropyltrimethoxysilane, N,N-dimethylaminopropyltrimethoxysilane and N,N-dimethylaminoethyltrimethoxysilane;
silanes containing a haloalkyl group, for instance 3-chloropropyltrimethoxysilane and trifluoropropyltrimethoxysilane;
silanes containing a glyceroxy group, for instance 3-glyceroxypropyltrimethoxysilane and bis(3-glyceroxypropyl)dimethoxysilane;
silanes containing a ureido group, for instance 3-ureidopropyltrimethoxysilane, 3-ureidopropylmethyldimethoxysilane and 3-ureidopropyldimethylmethoxysilane;
silanes containing a cyano group, for instance cyanopropyltrimethoxysilane, cyanopropylmethyldimethoxysilane and cyanopropyldimethylmethoxysilane.

Preferably, the compound of formula (IV) comprising a reactive organic group R is chosen from silanes containing an epoxy group, silanes containing a (meth)acryloxy group, silanes containing an alkenyl group, silanes containing a mercapto group and silanes containing an aminoalkyl group.

Examples of compounds (III) and (IV) that are preferred for the implementation of this invention are, respectively, tetraethoxysilane and methyltrimethoxysilane.

Hydrolysis and polymerization catalysts that may be used, independently, include basic catalysts such as sodium hydroxide, potassium hydroxide, sodium carbonate, sodium hydrogen carbonate and aqueous ammonia, or amines such as trimethylamine, triethylamine or tetramethylammonium hydroxide, or acidic catalysts such as organic acids, for instance, citric acid, acetic acid, methanesulfonic acid, p-toluenesulfonic acid, dodecylbenzenesulfonic acid or dodecylsulfonic acid, or mineral acids such as hydrochloric acid, sulfuric acid or phosphoric acid.

When it is present, the surfactant used is preferably a nonionic or anionic surfactant or a mixture thereof. Sodium dodecylbenzenesulfonate may be used as anionic surfactant. The end of the hydrolysis is marked by the disappearance of the water-insoluble products (III) and (IV) and the production of a homogeneous liquid layer.

The condensation step (b) may use the same catalyst as the hydrolysis step or another catalyst chosen from those mentioned above.

After this process, a suspension in water of fine organosilicone particles is obtained, which may then optionally be separated from their medium. The process described above may thus comprise an additional filtration step, for example on a membrane filter, of the product resulting from step (b), optionally followed by a step of centrifugation of the filtrate intended to separate the particles from the liquid medium, and then a step of drying the particles. Other separation methods may obviously be used.

The form of the hollow sphere portions obtained according to the above process, and the sizes thereof, will depend especially on the mode of contact of the products of step (b).

A rather basic pH and cold introduction of the polymerization catalyst into the mixture obtained from step (a) will lead to "bowl"-shaped hollow sphere portions with a rounded bottom, whereas a rather acidic pH and dropwise introduction of the mixture obtained from step (a) into the hot polymerization catalyst will lead to hollow sphere portions with a "horseshoe"-shaped cross section.

According to one preferred embodiment of the invention, "bowl"-shaped hollow sphere portions are used. These may be obtained as described in patent application JP-A-2003-128 788 or FR 2 902 654.

Horseshoe-shaped hollow sphere portions are described in patent application JP-A-2000-191 789.

Figure 1 of FR 2 902 654 illustrates a concave particle in the form of sphere portions with a bowl-shaped cross section. The width W2 corresponds to the diameter of the particles.

As emerges from this figure, these concave portions are formed (in a section perpendicular to a plane of the aperture delimited by the hollow sphere portion) of a small inner arc (11), a large outer arc (21) and segments (31) that connect the ends of the respective arcs, the width (W1) between the two ends of the small inner arc (11) ranging from 0.01 to 8 µm and preferably from 0.02 to 6 µm on average, the width (W2) between the two ends of the large outer arc (21) ranging from 0.05 to 10 µm and preferably from 0.06 to 8 µm on average and the height (H) of the large outer arc (21) ranging from 0.015 to 8 µm and preferably from 0.03 to 6 µm on average.

The sizes mentioned above are obtained by calculating the mean of the sizes of one hundred particles chosen from an image obtained using a scanning electron microscope.

As concave particles in the form of sphere portions that may be used according to the invention, examples that may be mentioned include:
bowl-shaped particles consisting of the crosslinked organosilicone Tak-110 (methylsilanol/silicate crosslinked polymer) from the company Takemoto Oil & Fat, of width 2.5 µm, height 1.2 µm and thickness 150 nm (particles sold under the name NLK-506 by the company Takemoto Oil & Fat);
bowl-shaped particles consisting of the crosslinked organosilicone Tak-110 (methylsilanol/silicate crosslinked polymer) from the company Takemoto Oil & Fat, of width 0.8 µm, height 0.4 µm and thickness 130 nm (particles sold under the name NLK-515 by the company Takemoto Oil & Fat);
bowl-shaped particles consisting of the crosslinked organosilicone Tak-110 (methylsilanol/silicate crosslinked polymer) from the company Takemoto Oil & Fat, of width 7 µm, height 3.5 µm and thickness 200 nm (particles sold under the name NLK-510 by the company Takemoto Oil & Fat).

These particles comprise or even consist of methylsilanol/silicate crosspolymer.

Advantageously, the concave silicone particles, in particular the bowls, have a mean diameter of less than or equal to 5 µm, especially ranging from 0.1 µm to 5 µm, preferentially ranging from 0.2 to 5 µm, more preferentially ranging from 0.5 to 4 µm and even more preferably ranging from 0.5 to 3 µm,

### Polytopes

According to another variant, the microparticles that may be used according to the invention are non-spherical fine particles in the form of polygons having at least six concave faces. These particles are characterized by a mean value of the maximum outside diameters of the said individual non-spherical fine particles ranging from 0.1 to 20 µm; a mean value of the ratio between the minimum outside diameters and the maximum outside diameters of the said individual non-spherical fine particles ranging from 0.60 to 0.97; and a mean number of concave surfaces, whose ratio of the relative maximum diameter to the maximum outside diameter ranges from 0.50 to 0.90, ranging from 6 to 14 per non-spherical fine particle.

These particles are described in greater detail in patent application EP 2 476 719 A1.

The non-spherical fine particles in the form of polygons having at least six concave faces that may be used according to the invention are also known as "polytopes" or "osselets", and the largest dimension of these particles preferably ranges from 1 to 10 µm).

### Golf balls

As golf balls that may be used, mention may be made most particularly of organic silicone microparticles comprising a polysiloxane crosslinked structure, of spherical overall shape and having a multitude of dimples at their surface.

More particularly, the polysiloxane crosslinked structure is a structure in which the polysiloxane units form a three-dimensional network. Preferably, the said siloxane units comprise at least two types of unit chosen from the units of formula (1), and preferably comprise a unit of average formula (2); the said formulae being the following:

R¹ₘSiO_{(4-m)/2} (1)

R²ₙSiO_{(4-n)/2} (2)

in which R¹ and R² represent an organic group linked directly to the silicon atom via a carbon atom; m is an integer ranging from 0 to 3; n ranges from 0.78 to 0.95.

The units of formula (1) more particularly represent SiO_{4/2}, R¹SiO_{3/2}, R¹₂SiO_{2/2} and R¹₃SiO_{1/2} in which R¹, which may be identical or different, more particularly represent a hydrocarbon-based group such as an alkyl, cycloalkyl, aryl, alkylaryl or aralkyl group, the alkyl group containing from 1 to 4 carbon atoms such as methyl, ethyl or butyl, preferably methyl, and the aromatic group more particularly representing a phenyl.

The hydrocarbon-based groups may be substituted with an epoxy or glyceroxy group, a halogen atom, a ureido group, a cyano radical or an amino radical, and preferably a C₁-C₄ glycidoxyalkyl group such as 2-glycidoxyethyl or 3-glycidoxypropyl, C₁-C₄ 2-(glycidoxycarbonyl)alkyls such as 2-(glycidoxycarbonyl)ethyl or 2-(glycidoxycarbonyl)propyl, C₁-C₄ 2-(epoxycyclohexyl)alkyls such as 2-(3,4-epoxycyclohexyl)propyl; the radicals comprising epoxy radicals being preferred, along with 2-glycidoxyethyl and 3-glycidoxypropyl.

According to a first variant, in the case where R¹ represents an unsubstituted hydrocarbon-based group as described previously, the siloxane units represented by formula (1) may be chosen from 1) silicic anhydride, 2) a siloxane unit represented by R¹SiO_{3/2}, such as a methylsiloxane, ethylsiloxane, butylsiloxane or phenylsiloxane unit, 3) a siloxane unit represented by R¹₂SiO_{2/2}, such as a dimethylsiloxane, diethylsiloxane, dibutylsiloxane, methylphenylsiloxane or diphenylsiloxane unit, or 4) a siloxane unit represented by R¹₃SiO_{1/2}, such as a trimethylsiloxane, triethylsiloxane, tributylsiloxane, dimethylphenylsiloxane or diethylphenylsiloxane unit. Methylsiloxane, dimethylsiloxane and trimethylsiloxane units are preferred.

According to a second variant, in the case where R¹ represents a substituted hydrocarbon-based radical as described previously, the siloxane units represented by formula (1) may be advantageously chosen from 1) a siloxane unit represented by R¹SiO_{3/2}, such as a 3-glycidoxypropylsiloxane or 2-glycidoxyethylsiloxane unit, 2) a siloxane unit represented by R¹₂SiO_{2/2}, such as a 3-glycidoxypropylmethylsiloxane or 2-glycidoxyethylmethylsiloxane unit, or 3) a siloxane unit represented by R¹₃SiO_{1/2}, such as a 3-glycidoxypropyldimethylsiloxane or 2-glycidoxyethyldimethylsiloxane unit.

In the case where the polysiloxane crosslinked structure comprises siloxane units as described above, the average formula of the siloxane units is represented by the average formula (2) indicated previously. Preferably, n in formula (2) ranges from 0.78 to 0.95 and preferably from 0.80 to 0.90. For example, a crosslinked polysiloxane may consist of units 1) SiO_{4/2}, R¹SiO_{3/2}, R¹₂SiO_{2/2} and R¹₃SiO_{1/2}, 2) units SiO_{4/2}, R¹SiO_{3/2} and R¹₂SiO_{2/2}, or 3) SiO_{4/2} and R¹SiO_{3/2}, the preferred being crosslinked polysiloxanes comprising SiO_{4/2} and R¹SiO_{3/2}, in which the mole ratio SiO_{4/2} / R¹SiO_{3/2} is between 5/95 and 22/78 and preferably between 10/90 and 20/80.

The organic silicone microparticles have, as indicated above, a spherical overall structure and a mean diameter ranging from 0.1 to 10 µm and preferably from 0.1 to 7 µm. It should be noted that the mean diameter is measured via the laser diffraction/scattering method.

As regards the dimples at the surface of the microparticles, their structure may be influenced by the proportion of the siloxane units. When they are viewed from the surface of the microparticle, they may be virtually circular, elliptic, in the form of wrinkles (wrinkle-like) or of irregular form, or even a combination of several forms. However, microparticles comprising at their surface a multitude of dimples of virtually circular structure are preferred (golfball appearance).

The silicone microparticles may be prepared according to several methods.

Preferably, the following two steps are performed:

### Step 1:

In this step, use is made of at least two compounds that are capable of forming silanol compounds, chosen from the compounds of formula (3) detailed later, with a mean value of the coefficient p of between 0.78 and 0.95.

These compounds are hydrolysed in the presence of a catalyst to produce the silanol compounds.

Formula (3) is as follows: R³ₐSiX₍₄₋ₚ₎, in which:
R³ is an organic group linked directly to the silicon atom via a silicon atom,
X represents a C₁-C₄ alkoxy group, an alkoxyethoxy group in which the alkoxy group is C₁-C₄, a C₂-C₄ acyloxy group, an N,N-dialkylamino group in which the alkyl radicals, which may be identical or different, are C₁-C₄, a hydroxyl group, a halogen atom or a hydrogen atom,
p is an integer ranging from 0 to 3.

These compounds of formula (3) form after the process of the units of structure (1). The compounds that are capable of forming silanols are advantageously represented by the compounds SiX₄, R³SiX₃, R³₂SiX₂ and R³₃SiX.

It should be noted that R³ has the same meaning as the radicals R¹ of formula (1).

X represents 1) a C₁-C₄ alkoxy group such as methoxy or ethoxy, 2) an alkoxyethoxy group with a C₁-C₄ alkoxy group, such as methoxyethoxy or butoxyethoxy, 3) a C₁-C₄ acyloxy group, such as acetoxy or propioxy, 4) an N,N-dialkylamino group with the alkyl radicals, which may be identical or different, being C₁-C₄, such as dimethylamino or diethylamino, 5) a hydroxyl group, 6) a halogen atom such as chlorine or bromine, or 7) a hydrogen atom.

According to a first variant, when R³ represents a hydrocarbon-based group (just like R¹), the compound that is capable of forming silanols (formula 3) may be chosen from 1) SiX₄, such as tetramethoxysilane, tetraethoxysilane, tetrabutoxysilane or tetrachlorosilane, 2) R³SiX₃, such as methyltrimethoxysilane, methyltriethoxysilane, methyltriacetoxysilane, phenyltrimethoxysilane, methyltris(dimethylamino)silane, methyltrichlorosilane, phenyltrichlorosilane, methyldichloromethoxysilane, methyldichlorohydrogensilane, methylsilanetriol, methyldichlorosilanol or methylchlorosilanediol, 3) R³₂SiX₂, such as dimethyldimethoxysilane, dimethyldiethoxysilane, methylphenyldimethoxysilane, dimethyldiacetoxysilane, dimethylbis(dimethylamino)silane, dimethyldichlorosilane, diethyldichlorosilane, diphenyldichlorosilane, dimethylchloromethoxysilane, methylethyldichlorosilane, dimethylsilanediol or diethylsilanediol, or 4) R³₃SiX, such as trimethylmethoxysilane, trimethylethoxysilane, dimethylethylmethoxysilane, trimethylacetoxysilane, trimethyl-(dimethylamino)silane, trimethylchlorosilane, triphenylchlorosilane or trimethylsilanol. The preferred compounds are chosen from compounds that finally form a methylsiloxane, dimethylsiloxane or trimethylsiloxane unit.

According to a second variant, when R³ represents a substituted hydrocarbon-based group (just like R¹), the compound that is capable of forming silanols (formula 3) may be chosen from 1) a silane comprising an epoxy group such as 3-glycidoxypropyltrimethoxysilane, 3-glycidoxypropyltriethoxysilane, 2-(3,4-epoxycyclohexyl)ethyltrimethoxysilane, 3-glycidoxypropylmethyldimethoxysilane, 2-glycidoxyethylmethyldimethoxysilane, 3-glycidoxypropyldimethylmethoxysilane or 2-glycidoxyethyldimethylmethoxysilane, 2) a silane comprising a glyceroxy group such as 3-glyceroxypropyltrimethoxysilane or bis(3-glyceroxypropyl)dimethoxysilane, 3) a silane comprising a haloalkyl group such as 3-chloropropyltrimethoxysilane or trifluoropropyltrimethoxysilane, 4) a silane comprising a ureido group such as 3-ureidopropyltrimethoxysilane, 3-ureidopropylmethyldimethoxysilane or 3-ureidopropyldimethylmethoxysilane, 5) a silane comprising a cyano group such as cyanopropyltrimethoxysilane, cyanopropylmethyldimethoxysilane or cyanopropyldimethylmethoxysilane, or 6) a silane comprising an N,N-dialkylamino group such as N,N-dimethylaminopropyltrimethoxysilane, N,N-dimethylaminopropylmethyldimethoxysilane or N,N-dimethylaminopropyldimethylmethoxysilane. The preferred compounds comprise an epoxy group.

In this first step, the compounds that are capable of forming silanols are chosen such that the value of p in the mean composition, i.e. in formula (3), is from 0.78 to 0.95 and preferably 0.80 to 0.90.

In addition, the said compounds are chosen more particularly from those comprising 1) SiX₄, R³SiX₃, R³₂SiX₂ and R³₃SiX, 2) those comprising SiX₄, R³SiX₃ and R³₂SiX₂, 3) those comprising SiX₄ and R³SiX₃; the preferred compounds being those comprising SiX₄, R³SiX₃, more particularly with a mole ratio SiX₄ / R³SiX₃ between 5/95 and 22/78 and preferably between 10/90 and 20/80.

During the first step, the hydrolysis catalyst used is chosen from those conventionally used in the field.

As examples of suitable catalysts, mention may be made of mineral acids such as hydrochloric acid, sulfuric acid and phosphoric acid, and also organic acids such as acetic acid, citric acid, methanesulfonic acid, para-toluenesulfonic acid, dodecylbenzenesulfonic acid and dodecylsulfonic acid.

Among the suitable catalysts, mention may also be made of mineral bases such as sodium hydroxide, potassium hydroxide, sodium carbonate and sodium bicarbonate and organic bases such as ammonia, trimethylamine, triethylamine, tetraethylammonium hydroxide, dodecyldimethylhydroxyethylammonium hydroxide and sodium methoxide.

During the first step, the compounds that are capable of forming silanols and the hydrolysis catalyst are mixed with water.

The weight ratio of water/compounds that are capable of forming silanols is between 10/90 and 70/30.

Advantageously, the content of hydrolysis catalyst is not more than 1% by weight relative to the total weight of compounds that are capable of forming silanols.

The reaction mixture may also comprise at least one surfactant that can facilitate the reaction of the compounds forming the silanols with water.

The first step may be performed by adding, in a single portion, the compounds that are capable of forming silanols, in water or by gradually adding the said compounds. If the compounds that are capable of forming silanols have very different reactivities, it is possible to hydrolyse them separately, sequentially, beginning with those having the slowest reactivity, and then adding those of higher reactivity and then continuing the hydrolysis.

The reaction temperature is such that the condensation of the silanol compounds formed during this first step is avoided. More particularly, the temperature is between 0 and 40°C and preferably not more than 30°C.

The silanols derived from the first step are used in the second step.

The reaction medium may be used as obtained or else pretreated before the second step in order to remove the hydrolysis catalyst, the unreacted reagents, or else treated to deactivate the remaining hydrolysis catalyst, for example by neutralization.

### Step 2:

The silanols derived from the first step are then condensed by placing them in contact with an aqueous solution comprising at least one condensation catalyst that is suitable for producing organic silicone microparticles.

The polycondensation catalyst may be chosen from the acidic or basic catalysts used during the first step.

During this step, the silanols derived from the first step and the polycondensation catalyst(s) may be mixed together in total or else gradually placed in contact. Preferably, the silanols derived from the preceding step are introduced gradually into a solution comprising the polycondensation catalyst(s).

Usually, the content of polycondensation catalyst(s) ranges between 1% and 40% by weight and preferably between 3% and 30% by weight relative to the silanols derived from the first step.

The temperature at which the second step is performed is generally at least 40°C and 100°C and is preferably between 60 and 95°C.

Silicone microparticles in the form of an aqueous suspension are then obtained.

After the reaction, the microparticles are separated from the aqueous reaction mixture and then dried.

For example, the reaction mixture may be passed through a filter (for example a membrane filter) and the recovered microparticles may then undergo centrifugation or filtration, especially under pressure. They are then advantageously dried at a temperature of between 100 and 250°C.

It is also possible to dry the reaction mixture directly at a temperature of between 100 and 250°C, for example by atomization.

The microparticles are preferably ground especially using a jet mill.

The microparticles of this type and processes for obtaining them are described especially in Japanese patent JP 38 46667 filed by Takemoto Oil & Fat.

As microparticles of "golfball" type that may be used according to the invention, examples that may be mentioned include the microparticles NLK800 and NLK801 from the company Takemoto Oil & Fat.

Preferably, the total amount of solid microparticles comprising at least one curved part and at least one breakage of curvature of the said curved part ranges from 1% to 10% and preferably from 2% to 7% by weight relative to the total weight of the emulsion.

### Rugby balls

As stated above, the O/O emulsion according to the invention may further comprise microparticles that comprise only one curvature. They may be of non-(hemi)spherical shape, in particular fusiform shape, also known as a "rugby ball" shape.

In the present text, a fusiform or "rugby ball" shape relates to a shape such as a sphere that is extended in one direction so as to have a major axis along which the fusiform particle has the longest diameter L₁ which is between approximately 0.05 µm and approximately 20 µm and two minor axes L₂ perpendicular to the major axis and along each of which the fusiform particle has the smallest diameter which is between approximately 0.03 µm and approximately 15 µm, L₁/L₂ being between approximately 1.1 and approximately 3.3.

The organosilicon material of crosslinked polysiloxane structure preferably comprises, or even is composed of, units of formula (I): SiO₂, and of formula (II): R¹SiO_{1.5}, in which R¹ represents an organic group containing a carbon atom directly linked to the silicon atom. The organic group may be a reactive organic group or an unreactive organic group, and preferably an unreactive organic group.

The organosilicon material of crosslinked polysiloxane structure also preferably comprises a first, a second and a third siloxane unit, which are, respectively, of formula (I): SiO₂, of formula (II): R¹SiO_{1.5} and of formula (III): R²R³SiO, in which R¹, R² and R³ are any identical or different organic groups, containing a carbon atom directly linked to a silicon atom; R² and R³ may independently be an unreactive organic group or an organic group not containing a reactive group or a reactive organic group or an organic group containing a reactive group. R² and/or R³ however preferably represent a reactive organic group or an organic group containing a reactive group.

The unreactive organic group may be a C₁-C₄ alkyl group, especially a methyl, ethyl, propyl or butyl group, or a phenyl group, and preferably a methyl group.

The reactive organic group may be an epoxy group, a (meth)acryloyloxy group, an alkenyl group, a mercaptoalkyl, aminoalkyl or haloalkyl group, a glyceroxy group, a ureido group or a cyano group. Preferably, the reactive organic group may be an epoxy group, a (meth)acryloyloxy group, an alkenyl group or a mercaptoalkyl or aminoalkyl group. The reactive organic group generally comprises from 2 to 6 carbon atoms and especially from 2 to 4 carbon atoms.

Epoxy groups that may be mentioned include a 2-glycidoxyethyl group, a 3-glycidoxypropyl group and a 2-(3,4-epoxycyclohexyl)propyl group.

(Meth)acryloyloxy groups that may be mentioned include a 3-methacryloyloxypropyl group and a 3-acryloyloxypropyl group.

Alkenyl groups that may be mentioned include vinyl, allyl and isopropenyl groups.

Mercaptoalkyl groups that may be mentioned include mercaptopropyl and mercaptoethyl groups.

Aminoalkyl groups that may be mentioned include a 3-[(2-aminoethyl)amino]propyl group, a 3-aminopropyl group and an N,N-dimethylaminopropyl group.

Haloalkyl groups that may be mentioned include a 3-chloropropyl group and a trifluoropropyl group.

Glyceroxy groups that may be mentioned include a 3-glyceroxypropyl group and a 2-glyceroxyethyl group.

A ureido group that may be mentioned is a 2-ureidoethyl group.

Cyano groups that may be mentioned include cyanopropyl and cyanoethyl groups.

Preferably, in the unit of formula (II), R¹ represents a methyl group.

Advantageously, the organosilicon material comprises the units (I) and (II) in a unit (I)/unit (II) mole ratio ranging from 30/70 to 50/50 and preferably ranging from 35/65 to 45/55.

Also advantageously, the organosilicon material comprises units (I), (II) and (III) such that the mole ratio of the molar sum of the first siloxane unit (I) and of the second unit (II) to the third siloxane unit (III) is approximately between 99/1 and 50/50 and more preferably between 90/10 and 60/40. The mole ratio of the first siloxane unit (I) relative to the second siloxane unit (II) may preferably be approximately between 23/77 and 40/60.

The organosilicon microparticles may especially be obtained via a process which comprises:
(a) the introduction into an aqueous medium, in the presence of at least one hydrolysis catalyst, and optionally of at least one surfactant, of a compound (IV) of formula SiX₄ and of a compound (V) of formula R⁴SiY₃, in which X and Y represent, independently of each other, a C₁-C₄ alkoxy group, an alkoxyethoxy group comprising a C₁-C₄ alkoxy group, a C₂-C₄ acyloxy group, an N,N-dialkylamino group comprising a C₁-C₄ alkyl group, a hydroxyl group, a halogen atom or a hydrogen atom, and R represents an organic group comprising a carbon atom directly bonded to the silicon atom; and
(b) the operation during which the mixture resulting from step (a) is placed in contact with an aqueous solution containing at least one polymerization catalyst and optionally at least one surfactant, at a temperature of between 30 and 85°C, for at least two hours.

Step (a) corresponds to a hydrolysis reaction and step (b) corresponds to a condensation reaction.

In step (a), the mole ratio between compound (IV) and compound (V) is generally in the range from 23/77 to 40/60, advantageously from 35/65 to 45/55 and is preferably 40/60.

The organosilicon particles may also be obtained via a process which comprises:
(a) the introduction into an aqueous medium, in the presence of at least one hydrolysis catalyst, of a silicon compounds forming a silanol group (IV) of formula SiX₄, a silicon compound forming a silanol group (V) of formula R⁴SiY₃ and a silicon compound forming a silanol group (VI) of formula R⁵R⁶SiZ₂, in a mole ratio (silicon compound forming a silanol group (IV) and silicon compound forming a silanol group (V))/silicon compound forming a silanol group (VI) from 99/1 to 50/50, preferably from 90/10 to 60/40, such that the silicon compounds forming a silanol group are hydrolysed to generate a silanol compound (in addition, the silicon compounds forming a silanol of formulae (IV) and (V) are preferably in a mole ratio from 23/77 to 40/60); and
(b) instigation of the condensation reaction of the silanol compound generated in an aqueous medium in the presence of at least one hydrolysis catalyst.

The weight ratio between water and the total of compounds (IV) and (V) is preferably in the range from 10/90 to 70/30. The order of introduction of compounds (IV) and (V) generally depends on their rate of hydrolysis. The temperature of the hydrolysis reaction is generally in the range from 0 to 40°C and generally does not exceed 30°C so as to avoid premature condensation of the compounds.

For the groups X, Y and Z of compounds (IV), (V) and (VI):
C₁-C₄ alkoxy groups that may be mentioned include methoxy and ethoxy groups;
alkoxyethoxy groups comprising a C₁-C₄ alkoxy group that may be mentioned include methoxyethoxy and butoxyethoxy groups;
C₂-C₄ acyloxy groups that may be mentioned include acetoxy and propionyloxy groups;
N,N-dialkylamino groups comprising a C₁-C₄ alkyl group that may be mentioned include dimethylamino and diethylamino groups;
halogen atoms that may be mentioned include chlorine and bromine atoms.

Compounds of formula (IV) that may be mentioned include tetramethoxysilane, tetraethoxysilane, tetrabutoxysilane, trimethoxyethoxysilane, tributoxyethoxysilane, tetraacetoxysilane, tetrapropioxysilane, tetra(dimethylamino)silane, tetra(diethylamino)silane, silane tetraol, chlorosilane triol, dichlorodisilanol, tetrachlorosilane and chlorotrihydrogenosilane. Preferably, the compound of formula (IV) is chosen from tetramethoxysilane, tetraethoxysilane and tetrabutoxysilane, and mixtures thereof.

The compound of formula (IV) leads, after the polymerization reaction, to the formation of the units of formula (I).

The compound of formula (V) leads, after the polymerization reaction, to the formation of the units of formula (II).

The group R in the compound of formula (V) has the meaning as described for the group R¹ of the compound of formula (II).

As examples of compounds of formula (V) comprising an unreactive organic group R, mention may be made of methyltrimethoxysilane, ethyltriethoxysilane, propyltributoxysilane, butyltributoxysilane, phenyltrimethoxyethoxysilane, methyltributoxyethoxysilane, methyltriacetoxysilane, methyltripropioxysilane, methyltri(dimethylamino)silane, methyltri(diethylamino)silane, methylsilanetriol, methylchlorodisilanol, methyltrichlorosilane or methyltrihydrogenosilane.

As examples of compounds of formula (V) comprising a reactive organic group R, mention may be made of:
silanes comprising an epoxy group, such as (3-glycidoxypropyl)trimethoxysilane, (3-glycidoxypropyl)triethoxysilane, [2-(3,4-epoxycyclohexyl)ethyl]trimethoxysilane, (3-glycidoxypropyl)methyldimethoxysilane, (2-glycidoxyethyl)methyldimethoxysilane, (3-glycidoxypropyl)dimethylmethoxysilane or (2-glycidoxyethyl)dimethylmethoxysilane;
silanes comprising a (meth)acryloyloxy group, such as (3-methacryloyloxypropyl)trimethoxysilane or (3-acryloyloxypropyl)trimethoxysilane;
silanes comprising an alkenyl group, such as vinyltrimethoxysilane, allyltrimethoxysilane or isopropenyltrimethoxysilane;
silanes comprising a mercapto group, such as mercaptopropyltrimethoxysilane or mercaptoethyltrimethoxysilane;
silanes comprising an aminoalkyl group, such as (3-aminopropyl)trimethoxysilane, (3-[(2-aminoethyl)amino]propyl)trimethoxysilane, (N,N-dimethylaminopropyl)trimethoxysilane or (N,N-dimethylaminoethyl)trimethoxysilane;
silanes comprising a haloalkyl group, such as (3-chloropropyl)trimethoxysilane or trifluoropropyltrimethoxysilane;
silanes comprising a glyceroxy group, such as (3-glyceroxypropyl)trimethoxysilane or bis(3-glyceroxypropyl)dimethoxysilane;
silanes comprising a ureido group, such as (3-ureidopropyl)trimethoxysilane, (3-ureidopropyl)methyldimethoxysilane or (3-ureidopropyl)dimethylmethoxysilane;
silanes comprising a cyano group, such as cyanopropyltrimethoxysilane, cyanopropylmethyldimethoxysilane or cyanopropyldimethylmethoxysilane.

Preferably, the compound of formula (V) comprising a reactive organic group R is chosen from silanes comprising an epoxy group, silanes comprising a (meth)acryloyloxy group, silanes comprising an alkenyl group, silanes comprising a mercapto group or silanes comprising an aminoalkyl group.

Examples of compounds (IV) and (V) that are preferred for the implementation of this invention are, respectively, tetraethoxysilane and methyltrimethoxysilane.

Hydrolysis and polymerization catalysts that may be used, independently, include basic catalysts such as sodium hydroxide, potassium hydroxide, sodium carbonate, sodium hydrogen carbonate or amines (such as ammonia, trimethylamine, triethylamine or tetramethylammonium hydroxide), or acidic catalysts chosen from organic acids, such as citric acid, acetic acid, methanesulfonic acid, p-toluenesulfonic acid, dodecylbenzenesulfonic acid or dodecylsulfonic acid, or mineral acids such as hydrochloric acid, sulfuric acid or phosphoric acid. When it is present, the surfactant used is preferably a nonionic or anionic surfactant or a mixture thereof. Sodium dodecylbenzenesulfonate may be used as anionic surfactant. The end of the hydrolysis is marked by the disappearance of the water-insoluble products (IV) and (V) and the production of a homogeneous liquid layer.

The silanol-forming silicon compound of formula (VI) forms the siloxane unit (III) as a result. The group Z in formula (VI) may be chosen from the groups listed for the group X in formula (IV). R⁵ and R⁶ in formula (VI) may be chosen from the groups described for R² and R³ in formula (III).

The condensation step (b) may use the same catalyst as the hydrolysis step or another catalyst chosen from those mentioned above.

At the end of this process, a suspension in water of organosilicon fine particles is obtained, the said particles optionally being able to be subsequently separated from their medium. The process described above may consequently comprise an additional filtration step, for example on a membrane filter, of the product resulting from step (b), optionally followed by a step of centrifugation of the filtrate intended to separate the microparticles from the liquid medium, and then a step of drying the particles. Other separation processes may obviously be used.

The fusiform microparticles may comprise slits along their major axes.

Moreover, the fusiform microparticles generally represent less than 40% by weight, preferably approximately between 1% by weight and 40% by weight, preferably approximately between 5% by weight and 20% by weight and more preferably approximately between 10% by weight and 15% by weight, of the powder composition.

According to a particular embodiment, the crosslinked polysiloxane comprises a first, a second and a third siloxane unit, which are, respectively, SiO₂, R¹SiO_{1.5} and R²R³SiO, in which R¹, R² and R³ are any identical or different organic groups, comprising a carbon atom directly linked to a silicon atom. The mole ratio of the third siloxane unit relative to the molar sum of the first, second and third siloxane units is approximately between 1% and 50%.

The mole ratio of the first siloxane unit relative to the second siloxane unit is approximately between 23/77 and 40/60. In the powder composition according to the present invention, R¹ and R² and/or R³ may preferably be chosen from the group consisting of the epoxy group, the (meth)acryloxy group, the mercaptoalkyl group, the aminoalkyl group and the organic groups comprising any of the preceding groups.

The fusiform microparticles and processes for obtaining them are described especially in Japanese patent applications 2003-171 465 or 2010-057419 (or European patent EP 2433979) filed by Takemoto Oil & Fat.

### Oils

The emulsion according to the invention comprises two oily phases, a first oily phase comprising at least a first oil chosen from silicone oils, hydrocarbon-based oils and fluoro oils, and a second oily phase comprising at least a second oil that is immiscible with the first oil(s), at room temperature and at atmospheric pressure (760 mmHg/1.013×10⁵ Pa).

For the purposes of the present invention, the term "immiscible oils" means that the mixing of these two oils does not lead to a homogeneous one-phase solution. The said mixing is performed with the same weight amount of each oil.

For the purposes of the invention, the term "oil" means a compound which is liquid at ambient temperature and ambient pressure, and preferably whose maximum viscosity is 200 000 cPs (200 Pa.s) at 25°C.

Also, and preferably, at least one of the oils is chosen from water-immiscible compounds (the evaluation is done with a protocol of test which is described later). In accordance with a particularly advantageous embodiment of the invention, the oil(s) are chosen from water-immiscible compounds.

It should be noted that the viscosities are measured according to the following protocol:
The viscosity is measured at 25°C ± 0.5°C using a Haake RS600 controlled-stress rheometer from the company Thermo Rheo equipped with a spindle of cone/plate geometry with a diameter of between 2 cm and 6 cm and an angle of between 1° and 2°, the choice of the spindle depending on the viscosity to be measured (the more fluid the formulation, the greater the diameter of the chosen cone and the smaller the angle).

The measurement is performed by applying on the oil sample a logarithmic ramp of shear gradient ε' ranging from 10⁻³ s⁻¹ to 1000 s⁻¹ for a duration of 5 minutes.

The rheogram representing the change in viscosity as a function of the shear gradient ε' is then plotted.

The value under consideration is that of the viscosity at 500 s⁻¹,whether it is measured at this gradient or extrapolated by the plot if no experimental point corresponds to this value.

More particularly, the oils are said to be "immiscible" when mixing them leads to a separation of phases according to the following protocols:
For oils whose viscosity is less than 10 000 cPs (10 Pa.s) at 25°C, the two oils to be evaluated are introduced (5 g/5 g) at room temperature into a conical-tipped plastic centrifuge tube (ref. Corning® 15mL PET Centrifuge Tubes, Rack Packed with Plug Seal Cap, Sterile (Product #430055) which is placed in a Vortex Genie 2 machine. Stirring is performed at speed 10 for 10 seconds, followed by manual inversion of the tube before replacing it in the Vortex machine. This cycle is repeated three times in succession. The mixture is then left to stand at room temperature for 48 hours.

If at least one of the oils has a viscosity of greater than or equal to 10 000 cPs (10 Pa.s) at 25°C, then the mixture of the two oils (5 g/5 g) is placed in an oven at 50°C for 30 minutes before performing the three stirring cycles described previously.

The mixture is then observed.

When the mixture is separated into two phases and the separation of the two phases is sharply delimited at the interface, the phases are said to be "separated" and the oils are consequently immiscible.

In the contrary case, the mixture is observed using a phase-contrast microscope, at room temperature (about 25°C). If a continuous phase and a dispersed phase in the form of drops is observed, the phases are said to be "separated" and the oils are considered as immiscible.

If the observation of the mixture reveals only a single phase, then the phases are said to be "non-separated" and the oils are considered as miscible.

This same protocol is used to check the miscibility of the oil with water, one of the oils being replaced by water.

More particularly, the composition according to the invention comprises at least a first oily phase containing at least one non-volatile oil, and a second oily phase containing at least one volatile or non-volatile oil.

Preferably, the first and second oily phases each contain at least one non-volatile oil.

The term "non-volatile" refers to an oil whose vapour pressure at room temperature (25°C) and atmospheric pressure is non-zero and is less than 10⁻³ mmHg (0.13 Pa).

The term "volatile" refers to an oil that can evaporate on contact with the skin in less than one hour, at room temperature and atmospheric pressure.

More particularly, the term "volatile oil" means an oil which has a non-zero vapour pressure, at room temperature (25°C) and atmospheric pressure, in particular having a vapour pressure ranging from 0.13 Pa to 40 000 Pa, preferably ranging from 1.3 Pa to 13 000 Pa and preferentially ranging from 1.3 Pa to 1300 Pa. 9.

A preferred embodiment of the invention is directed to an oil/oil emulsion in which the said non-volatile first oil or second oil, preferably the said first oil, is chosen from silicone oils and fluoro oils, or mixtures thereof, and more particularly from non-phenylated non-volatile silicone oils; phenylated non-volatile silicone oils, optionally bearing at least one dimethicone fragment; fluoro oils; or mixtures thereof.

Another preferred embodiment of the invention is directed an oil/oil emulsion in which the non-volatile first or second oil, preferably the second oil, is chosen from polar hydrocarbon-based non-volatile oils, in particular chosen from non-volatile oils comprising at least one free hydroxyl group or not comprising any, or from non-volatile oils comprising at least two free hydroxyl groups, or from apolar hydrocarbon-based non-volatile oils, or mixtures thereof.

### First oily phase

The first oily phase comprises at least a first non-volatile oil chosen from silicone oils, fluoro oils and hydrocarbon-based oils, or mixtures thereof, and more particularly, in accordance with a first embodiment, from non-phenylated non-volatile silicone oils; phenylated non-volatile silicone oils, optionally bearing at least one dimethicone fragment; fluoro oils; or mixtures thereof.

In accordance with a second embodiment, the first oily phase comprises at least a first hydrocarbon-based oil.

This first oily phase may be the continuous phase or the dispersed phase.

The term "silicone oil" means an oil containing at least one silicon atom, and in particular containing Si-O groups.

The term "polar hydrocarbon-based oil" means an oil formed essentially from, or even consisting of, carbon and hydrogen atoms, and also heteroatoms such as oxygen and nitrogen atoms, and not containing any silicon or fluorine atoms.

The term "fluoro oil" means an oil containing at least one fluorine atom.

### 1. Silicone oils

### Non-volatile non-phenylated silicone oils

The term "non-phenylated silicone oil" or "non-phenyl silicone oil" denotes a silicone oil which does not bear any phenyl substituents.

Representative examples of these non-volatile non-phenylated silicone oils which may be mentioned include polydimethylsiloxanes; alkyl dimethicones; vinylmethyl methicones; and also silicones modified with aliphatic groups and/or with functional groups such as hydroxyl, thiol and/or amine groups.

It should be noted that "dimethicone" (INCI name) corresponds to a polydimethylsiloxane (chemical name).

In particular, these oils may be chosen from the following non-volatile non-phenylated silicone oils:
- polydimethylsiloxanes (PDMSs),
- PDMSs comprising aliphatic groups, in particular alkyl or alkoxy groups, which are pendent and/or at the end of the silicone chain, these groups each comprising from 2 to 24 carbon atoms. By way of example, mention may be made of the cetyl dimethicone sold under the commercial reference Abil Wax 9801 from Evonik Goldschmidt,
- PDMSs comprising at least one aliphatic group and/or at least one functional group such as hydroxyl, thiol and/or amine groups,
- polysiloxanes modified with fatty acids, fatty alcohols or polyoxyalkylenes, and mixtures thereof.

The non-volatile non-phenylated silicone oil is preferably chosen from non-volatile dimethicone oils.

Preferably, these non-volatile non-phenylated silicone oils are chosen from polydimethylsiloxanes; alkyl dimethicones and also PDMSs comprising at least one aliphatic group, in particular C₂-C₂₄ alkyl groups, and/or at least one functional group such as hydroxyl, thiol and/or amine groups.

The non-phenylated silicone oil may be chosen in particular from silicones of formula (I'): 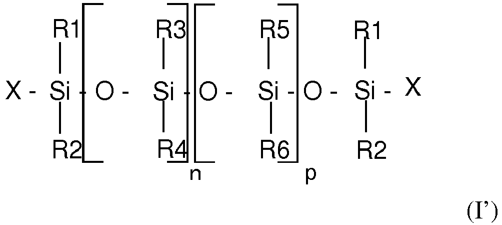 in which:
R₁, R₂, R₅ and R₆ are, together or separately, an alkyl radical containing 1 to 6 carbon atoms,
R₃ and R₄ are, together or separately, an alkyl radical containing from 1 to 6 carbon atoms, a vinyl radical, an amine radical or a hydroxyl radical,
X is an alkyl radical containing from 1 to 6 carbon atoms, a hydroxyl radical or an amine radical,
n and p are integers chosen so as to have a fluid compound, in particular whose viscosity at 25°C is between 9 centistokes (cSt) (9 x 10⁻⁶ m²/s) and 800 000 cSt (i.e. between 8 mPa.s and 720 000 mPa.s).

As non-volatile non-phenylated silicon oils which can be used according to the invention, mention may be made of those for which:
- the substituents R₁ to R₆ and X represent a methyl group, and p and n are such that the viscosity is 500 000 cSt (i.e. 450 000 mPa.s), for example the product sold under the name SE30 by the company General Electric, the product sold under the name AK 500000 by the company Wacker, the product sold under the name Mirasil DM 500 000 by the company Bluestar, and the product sold under the name Dow Corning 200 Fluid 500 000 cSt (i.e. 450 000 mPa.s) by the company Dow Corning,
- the substituents R₁ to R₆ and X represent a methyl group, and p and n are such that the viscosity is 60 000 cSt (54 000 mPa.s), for example the product sold under the name Dow Corning 200 Fluid 60 000 CS by the company Dow Corning, and the product sold under the name Wacker Belsil DM 60 000 by the company Wacker,
- the substituents R₁ to R₆ and X represent a methyl group, and p and n are such that the viscosity is 100 cSt (i.e. 90 mPa.s) or 350 cSt (i.e. 315 mPa.s), for example the products sold respectively under the names Belsil DM100 and Dow Corning 200 Fluid 350 CS by the company Dow Corning,
- the substituents R₁ to R₆ represent a methyl group, the group X represents a hydroxyl group, and n and p are such that the viscosity is 700 cSt (630 mPa.s), for example the product sold under the name Baysilone Fluid T0.7 by the company Momentive.

### Non-volatile phenylated silicone oils

The expression "phenylated silicone oil" or "phenyl silicone oil" denotes a silicone oil bearing at least one phenyl substituent.

These phenylated silicone oils may be chosen from those which also bear at least one dimethicone fragment, or from those which do not bear one.

According to the invention, a dimethicone fragment corresponds to the following unit:

-Si(CH₃)₂-O-.

The non-volatile phenylated silicone oil may thus be chosen from:
**a)** phenyl silicone oils optionally bearing a dimethicone fragment corresponding to the following formula (I): 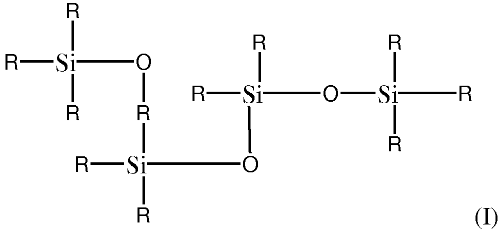 in which the groups R, which are monovalent or divalent, represent, independently of each other, a methyl or a phenyl, with the proviso that at least one group R represents a phenyl.
   Preferably, in this formula, the phenyl silicone oil comprises at least three, for example at least four, at least five or at least six, phenyl groups.
**b)** phenyl silicone oils optionally bearing a dimethicone fragment corresponding to the following formula (II): 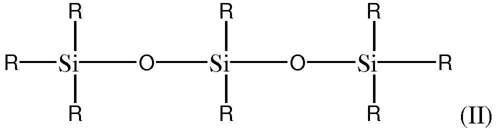 in which the groups R represent, independently of each other, a methyl or a phenyl, with the proviso that at least one group R represents a phenyl.

Preferably, in this formula, the compound of formula (II) comprises at least three, for example at least four or at least five, phenyl groups.

Mixtures of different phenylorganopolysiloxane compounds described above can be used.

Examples which may be mentioned include mixtures of triphenyl-, tetraphenyl-or pentaphenylorganopolysiloxanes.

Among the compounds of formula (II), mention may more particularly be made of phenyl silicone oils which do not bear a dimethicone fragment, corresponding to formula (II) in which at least 4 or at least 5 radicals R represent a phenyl radical, the remaining radicals representing methyls.

Such non-volatile phenyl silicone oils are preferably trimethylpentaphenyltrisiloxane or tetramethyltetraphenyltrisiloxane. They are in particular sold by Dow Corning under the reference PH-1555 HRI or Dow Corning 555 Cosmetic Fluid (chemical name: 1,3,5-trimethyl-1,1,3,5,5-pentaphenyltrisiloxane; INCI name: trimethylpentaphenyltrisiloxane), or the tetramethyltetraphenyltrisiloxane sold under the reference Dow Corning 554 Cosmetic Fluid by Dow Corning can also be used.

They correspond in particular to the following formulae (III), (III'): 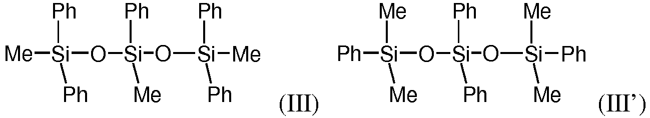 in which Me represents methyl, and Ph represents phenyl.
**c)** phenyl silicone oils bearing at least one dimethicone fragment corresponding to the following formula (IV): 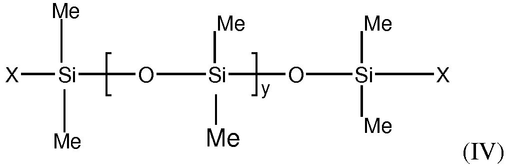 in which Me represents methyl, y is between 1 and 1000 and X represents -CH₂-CH(CH₃)(Ph).
**d)** phenyl silicone oils corresponding to formula (V) below, and mixtures thereof: 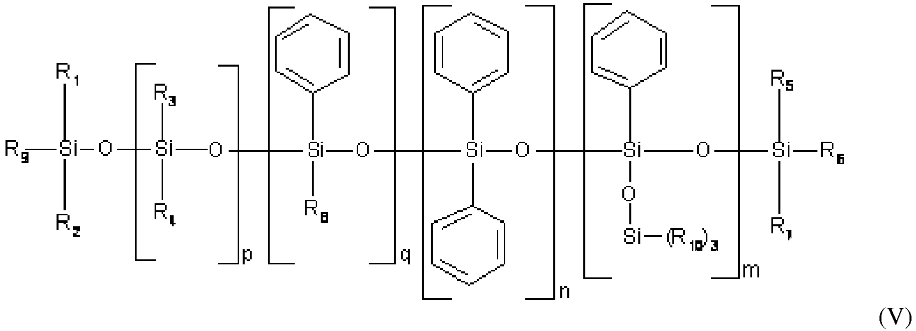 in which:
   - R₁ to R₁₀, independently of each other, are saturated or unsaturated, linear, cyclic or branched, preferably saturated or unsaturated, linear or branched, C₁-C₃₀ hydrocarbon-based radicals,
   - m, n, p and q are, independently of each other, integers between 0 and 900, with the proviso that the sum m+n+q is other than 0.

Preferably, the sum m+n+q is between 1 and 100. Preferably, the sum m+n+p+q is between 1 and 900 and preferably between 1 and 800. Preferably, q is equal to 0.

Preferably, R₁ to R₁₀, independently of each other, represent a linear or branched C₁-C₃₀ alkyl radical, preferably C₁-C₂₀ and more particularly C₁-C₁₆ alkyl, or a monocyclic or polycyclic C₆-C₁₄ and in particular C₁₀-C₁₃ aryl radical, or an aralkyl radical, the alkyl part of which is preferably C₁-C₃ alkyl.

Preferably, R₁ to R₁₀ may each represent a methyl, ethyl, propyl, butyl, isopropyl, decyl, dodecyl or octadecyl radical, or alternatively a phenyl, tolyl, benzyl or phenethyl radical. R₁ to R₁₀ may in particular be identical, and in addition may be a methyl radical.

According to a first more particular embodiment of formula (V), mention may be made of:
**i)** phenyl silicone oils optionally bearing at least one dimethicone fragment corresponding to formula (VI) below, and mixtures thereof: 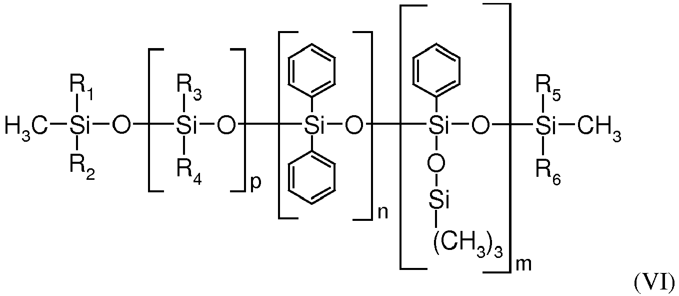 in which:
   - R₁ to R₆, independently of each other, are saturated or unsaturated, linear, cyclic or branched, preferably saturated or unsaturated, linear or branched, C₁-C₃₀ hydrocarbon-based radicals, a preferably C₆-C₁₄ aryl radical or an aralkyl radical, the alkyl part of which is C₁-C₃ alkyl,
   - m, n and p are, independently of each other, integers between 0 and 100, with the proviso that the sum n+m is between 1 and 100.

   Preferably, R₁ to R₆, independently of each other, represent a C₁-C₃₀, preferably C₁-C₂₀ and in particular C₁-C₁₆, alkyl radical, or a C₆-C₁₄ aryl radical which is monocyclic (preferably C₆) or polycyclic and in particular C₁₀-C₁₃, or an aralkyl radical (preferably the aryl part is C₆ aryl; the alkyl part is C₁-C₃ alkyl).
   Preferably, R₁ to R₆ may each represent a methyl, ethyl, propyl, butyl, isopropyl, decyl, dodecyl or octadecyl radical, or alternatively a phenyl, tolyl, benzyl or phenethyl radical.
   R₁ to R₆ may in particular be identical, and in addition may be a methyl radical. Preferably, m = 1 or 2 or 3, and/or n = 0 and/or p = 0 or 1 can be applied, in formula (VI).
   According to one particular embodiment, the non-volatile phenylated silicone oil is chosen from phenylated silicone oils having at least one dimethicone fragment.
   Preferably, such oils correspond to compounds of formula (VI) in which:
   **A)** m=0 and n and p are, independently of each other, integers between 1 and 100.
      Preferably, R₁ to R₆ are methyl radicals.
      According to this embodiment, the silicone oil is preferably chosen from a diphenyl dimethicone such as KF-54 from Shin Etsu, KF54HV from Shin Etsu, KF-50-300CS from Shin Etsu, KF-53 from Shin Etsu or KF-50-100CS from Shin Etsu.
   **B)** p is between 1 and 100, the sum n+m is between 1 and 100, and n=0.

   These phenyl silicone oils optionally bearing at least one dimethicone fragment correspond more particularly to formula (VII) below: 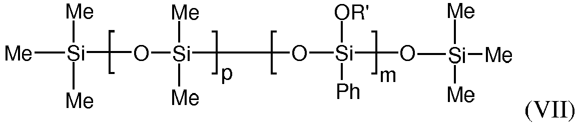 in which Me is methyl and Ph is phenyl, OR' represents a group -OSiMe₃ and p is 0 or is between 1 and 1000, and m is between 1 and 1000. In particular, m and p are such that the compound (VII) is a non-volatile oil.
   According to a first embodiment of non-volatile phenylated silicone bearing at least one dimethicone fragment, p is between 1 and 1000 and m is more particularly such that the compound (VII) is a non-volatile oil. Trimethylsiloxyphenyldimethicone, sold in particular under the reference Belsil PDM 1000 by the company Wacker, may, for example, be used.
   According to a second embodiment of non-volatile phenylated silicone not bearing a dimethicone fragment, p is equal to 0 and m is between 1 and 1000, and in particular is such that the compound (VII) is a non-volatile oil.
   Phenyltrimethylsiloxytrisiloxane, sold in particular under the reference Dow Corning 556 Cosmetic Grade Fluid (DC556), may, for example, be used.
**ii)** non-volatile phenyl silicone oils not bearing a dimethicone fragment corresponding to formula (VIII) below, and mixtures thereof: 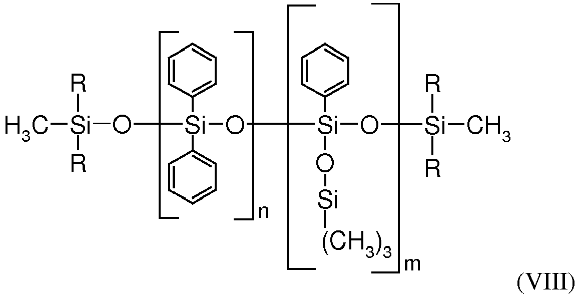 in which:
   - R groups (Rs), independently of each other, represent a saturated or unsaturated, linear, cyclic or branched, preferably saturated or unsaturated, linear or branched, C₁-C₃₀ hydrocarbon-based radical, more particularly, Rs represent a C₁-C₃₀ alkyl radical, an aryl radical, preferably a C₆-C₁₄ aryl radical, or an aralkyl radical, the alkyl part of which is C₁-C₃ alkyl,
   - m and n are, independently of each other, integers between 0 and 100, with the proviso that the sum n+m is between 1 and 100.

Preferably, R groups, independently of each other, represent a linear or branched C₁-C₃₀ and in particular a C₁-C₂₀, in particular C₁-C₁₆ alkyl radical, a monocyclic or polycyclic C₆-C₁₄, and in particular C₁₀-C₁₃, aryl radical, or an aralkyl radical of which preferably the aryl part is C₆ aryl and the alkyl part is C₁-C₃ alkyl.

Preferably, the R groups may each represent a methyl, ethyl, propyl, butyl, isopropyl, decyl, dodecyl or octadecyl radical, or alternatively a phenyl, tolyl, benzyl or phenethyl radical.

The R groups may in particular be identical, and in addition may be a methyl radical.

Preferably, m = 1 or 2 or 3, and/or n = 0 and/or p = 0 or 1 can be applied, in formula (VIII).

According to one preferred embodiment, n is an integer between 0 and 100 and m is an integer between 1 and 100, with the proviso that the sum n+m is between 1 and 100, in formula (VIII). Preferably, R is a methyl radical.

According to one embodiment, a phenyl silicone oil of formula (VIII) with a viscosity at 25°C of between 5 and 1500 mm²/s (i.e. 5 to 1500 cSt), and preferably with a viscosity of between 5 and 1000 mm²/s (i.e. 5 to 1000 cSt), may be used.

According to this embodiment, the non-volatile phenyl silicone oil is preferably chosen from phenyl trimethicones (when n=0) such as DC556 from Dow Corning (22.5 cSt), or else from diphenylsiloxyphenyl trimethicone oil (when m and n are between 1 and 100) such as KF56 A from Shin Etsu, or the Silbione 70663V30 oil from Rhône-Poulenc (28 cSt). The values in parentheses represent the viscosities at 25°C.
**e)** phenyl silicone oils optionally bearing at least one dimethicone fragment corresponding to the following formula, and mixtures thereof: 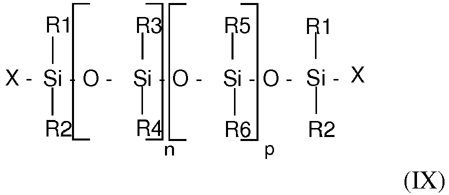 in which:
   R₁, R₂, R₅ and R₆, which may be identical or different, are an alkyl radical containing 1 to 6 carbon atoms,
   R₃ and R₄, which may be identical or different, are an alkyl radical containing from 1 to 6 carbon atoms or an aryl radical (preferably C₆-C₁₄), with the proviso that at least one of R₃ and R₄ is a phenyl radical,
   X is an alkyl radical containing from 1 to 6 carbon atoms, a hydroxyl radical or a vinyl radical,
   n and p being an integer greater than or equal to 1, chosen so as to give the oil a weight-average molecular weight of less than 200 000 g/mol, preferably less than 150 000 g/mol and more preferably less than 100 000 g/mol.
**f)** and a mixture thereof.

### 2. Fluoro oils

According to another embodiment, the first non-volatile oil is chosen from fluoro oils. The fluoro oils that may be used according to the invention may be chosen from fluorosilicone oils, fluoro polyethers and fluorosilicones especially as described in document EP-A-847 752, and perfluoro compounds.

According to the invention, the term "perfluoro compounds" is intended to mean compounds in which all the hydrogen atoms have been replaced with fluorine atoms.

According to a preferred embodiment, the first fluoro oil according to the invention is chosen from perfluoro oils.

As examples of perfluoro oils that may be used in the invention, mention may be made of perfluorodecalins and perfluoroperhydrophenanthrenes.

According to one preferred embodiment, the fluoro oil is chosen from perfluoroperhydrophenanthrenes, and in particular the Fiflow® products sold by the company Créations Couleurs. In particular, use may be made of the fluoro oil of which the INCI name is Perfluoroperhydrophenanthrene, sold under the reference Fiflow 220 by the company F2 Chemicals.

Preferably, the first oily phase comprises at least one first non-volatile oil chosen from the non-phenylated oils of formula (I), the phenylated oils of formula (II), especially (III), of formula (V), in particular (VI) or (VII), and also mixtures thereof.

### 3. Hydrocarbon-based oils

As indicated previously, a second embodiment consists in using, as first oil phase, at least one hydrocarbon-based oil, chosen in particular from non-volatile oils comprising not more than one free hydroxyl group or not comprising any, or from non-volatile oils comprising at least two free hydroxyl groups, or from apolar hydrocarbon-based non-volatile oils, or mixtures thereof.

These oils will be described in greater detail during the description of the second oily phase, and reference may be made thereto.

### Second oily phase

The second oily phase comprises at least one second non-volatile or volatile oil, which is immiscible with the first oil, at room temperature.

Preferably, the second oily phase comprises at least one second non-volatile oil, which is immiscible with the first oil(s), at room temperature.

This second oily phase may be the continuous phase or the dispersed phase.

The second oil(s) may advantageously be chosen from polar hydrocarbon-based non-volatile oils, in particular chosen from non-volatile oils comprising at most one free hydroxyl group or not comprising any, or from non-volatile oils comprising at least two free hydroxyl groups, or from apolar hydrocarbon-based non-volatile oils, or mixtures thereof. According to a second possibility, the second oil(s) are chosen from silicone oils that are immiscible with the first oil(s). The non-volatile silicone oils listed in the context of the definition of the first oily phase may be used as oil(s) of the second oily phase. Their description will not be repeated in this part of the text and reference may be made thereto, most particularly for the preferred silicones.

### 1. Polar non-volatile hydrocarbon-based oils

The term "polar hydrocarbon-based oil" means an oil formed essentially from, or even consisting of, carbon and hydrogen atoms, and also heteroatoms such as oxygen and nitrogen atoms, and not containing any silicon or fluorine atoms.

It may thus contain alcohol, ester, ether, carboxylic acid, amine and/or amide groups.

In particular, the hydrocarbon-based non-volatile polar oil may be chosen from the list of oils below, and mixtures thereof:
a) non-volatile oils comprising not more than one free hydroxyl group or not comprising any

The second oil(s) may be chosen from non-volatile hydrocarbon-based oils comprising not more than one free hydroxyl group, or not comprising any. As examples of oils of this type, mention may be made of:
**i)** Ester oils
   * Hydrocarbon-based plant oils such as liquid triglycerides of fatty acids containing from 4 to 40 carbon atoms and more particularly from 4 to 24 carbon atoms.
      Examples that may be mentioned include heptanoic or octanoic acid triglycerides, jojoba oil, sesame oil and ximenia seed oil, or mixtures thereof.
   * Synthetic glycerides such as those of capric/caprylic acids, C18-36 acid triglyceride (Dub TGI 24 from Stéarinerie Dubois).
   * Monoesters or diesters obtained from a saturated or unsaturated, aromatic or non-aromatic monocarboxylic or dicarboxylic fatty acid, in particular comprising from 4 to 40 and in particular from 4 to 24 carbon atoms, optionally comprising a free hydroxyl, on the one hand, and from a saturated or unsaturated, aromatic or non-aromatic monoalcohol or polyol, comprising from 2 to 40 and in particular from 3 to 24 carbon atoms, on the other hand; the number of carbon atoms (excluding the carbonyl group) being at least 12 and preferably at least 16, the ester comprising at most one free hydroxyl, if it contains any.
      - As examples of monoesters or diesters, mention may be made of purcellin oil (cetostearyl octanoate), isononyl isononanoate, C₁₂ to C₁₈ alkyl benzoate such as 2-octyldodecyl benzoate, 2-ethylhexyl palmitate, octyldodecyl neopentanoate, 2-octyldodecyl stearate, 2-octyldodecyl erucate, oleyl erucate, isostearyl isostearate, alcohol or polyalcohol, preferably diol, octanoates, decanoates or ricinoleates, isopropyl myristate, isopropyl palmitate, butyl stearate, hexyl laurate, 2-ethylhexyl palmitate, 2-hexyldecyl laurate, 2-octyldecyl palmitate, 2-octyldodecyl myristate and 2-diethylhexyl succinate; or mixtures thereof.
      - Fatty acid monoesters and diesters, in particular of C₄-C₂₂ and preferably C₆-C₂₂, and especially of octanoic acid, heptanoic acid, lanolic acid, oleic acid, lauric acid or stearic acid, and of C₃-C₆ glycol, for instance propylene glycol dioctanoate, propylene glycol monoisostearate or neopentyl glycol diheptanoate, are also suitable for use.
      - Hydroxylated monoesters and diesters, preferably with a total carbon number ranging from 20 to 70, for instance isostearyl lactate, octyl hydroxystearate, octyldodecyl hydroxystearate or diisostearyl malate.
      - C₁-C₄ monoesters of N-acylated amino acids, such as for instance those of formula R₁CONR₂CHR₃(CH₂)ₙCOOR₄ wherein R₁ represents a C₅-C₂₁ alkyl group, R₂, R₃ and R₄ same or different, represent a C₁-C₄ alkyl group, R₃ possibly being a hydrogen. For instance, isopropryl sarcosinate lauroyl may be cited,
   * Polyesters comprising at least three ester functions, of saturated, unsaturated or aromatic, linear, branched or cyclic, optionally hydroxylated, C₄-C₄₀ monocarboxylic or polycarboxylic acids and, respectively, of C₂-C₄₀ and preferably C₃-C₄₀ polyols or monoalcohols; the said polyester optionally comprising at least one free hydroxyl.

   By way of example, mention may be made of oils comprising three ester functions, of an acid comprising three monohydroxylated carboxylic functions, and of a C₂-C₄ monoalcohol, in particular triethyl citrate.
   By way of example, mention may be made of linear fatty acid esters with a total carbon number ranging from 35 to 70, for instance pentaerythrityl tetrapelargonate (MW = 697 g/mol).
   Esters of branched fatty alcohols or of branched fatty acids, for instance, especially, triisoarachidyl citrate (MW = 1033.76 g/mol), pentaerythrityl tetraisononanoate (MW = 697 g/mol), glyceryl triisostearate (MM = 891 g/mol), pentaerythrityl tetraisostearate (MW = 1202 g/mol), poly(2-glyceryl) tetraisostearate (MW = 1232 g/mol), and also those described in patent application EP-A-0 955 039, for instance glyceryl tris(2-decyl)tetradecanoate (MW = 1143 g/mol) or pentaerythrityl tetrakis(2-decyl)tetradecanoate (MW = 1538 g/mol), are also suitable for use.
   Mention may also be made of esters of aromatic acids and of alcohols comprising 4 to 22 atoms, such as tridecyl trimellitate (MW = 757 g/mol).
   Use may also be made of polyesters resulting from the esterification of at least one hydroxylated carboxylic acid triglyceride with an aliphatic monocarboxylic acid and with an aliphatic dicarboxylic acid, which is optionally unsaturated, for instance the succinic acid and isostearic acid castor oil sold under the reference Zenigloss by Zenitech;
**ii)** saturated or unsaturated, linear or branched monohydroxylated fatty alcohols containing from 8 to 30 carbon atoms and more advantageously from 12 to 26 carbon atoms, for instance octyldodecanol, 2-butyloctanol, 2-hexyldecanol, 2-undecylpentadecanol or oleyl alcohol;
**iii)** saturated or unsaturated C₁₂-C₂₆ and preferably C₁₂-C₂₂ fatty acids, such as oleic acid, linoleic acid and linolenic acid, and mixtures thereof;
**iv)** dialkyl carbonates, the two alkyl chains possibly being identical or different, such as dicaprylyl carbonate sold under the name Cetiol CC® by Cognis; and
**v)** vinylpyrrolidone copolymers such as the vinylpyrrolidone/1-hexadecene copolymer, Antaron V-216 sold or manufactured by the company ISP (MW = 7300 g/mol).

### b. Non-volatile oils comprising at least two free hydroxyl groups:

The second oil(s) may be chosen from non-volatile hydrocarbon-based oils comprising at least two free hydroxyl groups and preferably at least three free hydroxyl groups.

According to a first advantageous variant of the invention, the second oil(s) also comprise at least one ester function.

Examples of suitable oils that may be mentioned include:
* hydrocarbon-based plant oils such as liquid triglycerides of fatty acids containing from 4 to 40 carbon atoms and comprising at least two free hydroxyl groups and advantageously at least three free hydroxyl groups, for instance castor oil;
* hydroxylated esters, preferably with a total carbon number ranging from 35 to 70, for instance poly(2-glyceryl) triisostearate (MW = 965 g/mol), poly(2-glyceryl) isostearate; poly(2-glyceryl) diisostearate; poly(3-glyceryl) diisostearate, glyceryl stearate; glyceryl isostearate; or mixtures thereof;
* esters of a diol dimer and of a diacid dimer of general formula

   HO-R¹-(-OCO-R²-COO-R¹-)ₕ-OH,

   in which:
   R¹ represents a diol dimer residue obtained by hydrogenation of dilinoleic diacid,
   R² represents a hydrogenated dilinoleic diacid residue, and
   h represents an integer ranging from 1 to 9,
   especially the esters of dilinoleic diacids and of dilinoleyl diol dimers sold by the company Nippon Fine Chemical under the trade names Lusplan DD-DA5® and DD-DA7®;
* polyesters obtained by condensation of an unsaturated fatty acid dimer and/or trimer and of diol, in particular such as of dilinoleic acid and of 1,4-butanediol. Mention may especially be made in this respect of the polymer sold by Biosynthis under the name Viscoplast 14436H (INCI name: dilinoleic acid/butanediol copolymer), or else copolymers of polyols and of dimer diacids, and esters thereof, such as Hailucent ISDA.

According to a second advantageous variant of the invention, the second hydrocarbon -based oil(s) are chosen from polyhydroxylated alcohols, preferably of C₂-C₈ and more preferably of C₃-C₆, comprising two to three hydroxyl groups, such as glycerol, propylene glycol, pentylene glycol, 1,3-butylene glycol, dipropylene glycol or diglycerol, and a mixture thereof.

### 2. Apolar non-volatile hydrocarbon-based oils

The composition according to the invention may also comprise, as oil(s) present in the second oily phase, at least one apolar non-volatile hydrocarbon-based oil.

These oils may be of plant, mineral or synthetic origin.

For the purposes of the present invention, the term "apolar oil" means an oil formed essentially from, or even consisting of, carbon and hydrogen atoms, and not containing any oxygen, nitrogen, silicon or fluorine atoms.

Preferably, the non-polar non-volatile hydrocarbon-based oil may be chosen from linear or branched hydrocarbons of mineral or synthetic origin, such as:
- liquid paraffin or derivatives thereof,
- squalane,
- isoeicosane,
- naphthalene oil,
- polybutylenes such as Indopol H-100 (molar mass or MW = 965 g/mol), Indopol H-300 (MW = 1340 g/mol) and Indopol H-1500 (MW = 2160 g/mol) sold or manufactured by the company Amoco,
- polyisobutenes,
- hydrogenated polyisobutylenes such as Parleam® sold by the company Nippon Oil Fats, Panalane H-300 E sold or manufactured by the company Amoco (MW = 1340 g/mol), Viseal 20000 sold or manufactured by the company Synteal (MW = 6000 g/mol) and Rewopal PIB 1000 sold or manufactured by the company Witco (MW = 1000 g/mol), or alternatively Parleam Lite sold by NOF Corporation,
- decene/butene copolymers, polybutene/polyisobutene copolymers, in particular Indopol L-14,
- polydecenes and hydrogenated polydecenes such as: Puresyn 10 (MW = 723 g/mol) and Puresyn 150 (MW = 9200 g/mol) sold or manufactured by the company Mobil Chemicals, or alternatively Puresyn 6 sold by ExxonMobil Chemical),
- and mixtures thereof.

### 3. Volatile silicone or hydrocarbon-based oils

The composition according to the invention may also comprise, as oil(s) present in the second oily phase, at least one volatile silicone or hydrocarbon-based oil.

According to the invention, these volatile oils especially facilitate the application of the composition to the skin, the lips or the integuments.

These oils may be hydrocarbon-based oils or silicone oils optionally comprising alkyl or alkoxy groups that are pendent or at the end of the silicone chain, or a mixture of these oils.

As volatile silicone oils that may be used in the invention, mention may be made of linear or cyclic silicone oils with a viscosity at room temperature of less than 8 cSt and especially containing from 2 to 7 silicon atoms, these silicones optionally comprising alkyl or alkoxy groups containing from 1 to 10 carbon atoms. As volatile silicone oils that may be used in the invention, mention may be made especially of octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane and dodecamethylpentasiloxane, and mixtures thereof.

As volatile hydrocarbon-based oils that may be used in the invention, mention may be made of volatile hydrocarbon-based oils containing from 8 to 16 carbon atoms, and mixtures thereof, especially branched C₈-C₁₆ alkanes such as C₈-C₁₆ isoalkanes (also known as isoparaffins), isododecane, isodecane, isohexadecane and, for example, the oils sold under the trade names Isopar or Permethyl, and mixtures thereof.

Use is preferably made of isododecane (Permethyl 99 A), C₈-C₁₆ isoparaffins such as Isopar L, E, G or H, or mixtures thereof, optionally combined with decamethyltetrasiloxane or with cyclopentasiloxane.

Use may also be made of volatile fluoro oils.

The emulsion according to the invention more particularly comprises from 5% to 95% by weight and preferably from 30% to 70% by weight of the oil(s) of the first oily phase, relative to the weight of the emulsion.

The emulsion according to the invention more particularly comprises from 5% to 95% by weight and preferably from 30% to 70% by weight of the oil(s) of the second oily phase, relative to the weight of the emulsion.

Preferably, the second oily phase comprises at least one non-volatile oil.

According to this variant, the content of volatile oil of the second oily phase represents from 0.1% to 30% by weight relative to the weight of the emulsion.

In accordance with an advantageous embodiment of the invention, the weight ratio of the oil(s) of the first oily phase relative to the oil(s) of the second oily phase represents from 5/95 to 95/5 and preferably from 30/70 to 70/30.

According to a first embodiment of the invention, the composition comprises a first oily phase containing at least a first oil chosen from non-volatile silicone oils and a second oily phase containing at least a second oil chosen from non-volatile apolar hydrocarbon-based oils.

According to a particularly advantageous embodiment, non-volatile apolar oil(s) are chosen from hydrogenated or non hydrogenated polydecenes, hydrogenated or non hydrogenated polybutenes and their mixtures.

Preferably, the apolar oils are chosen from oils with a viscosity of at least 50 cPS, preferably 60 cPs.

According to a second embodiment of the invention, the composition comprises a first oily phase containing at least a first oil chosen from non-phenylated non-volatile silicone oils or phenylated non-volatile silicone oils bearing at least one dimethicone fragment, and a second oily phase containing at least a second oil chosen from non-volatile hydrocarbon-based oils comprising not more than one free hydroxyl group or not comprising any.

As regards the non-phenylated non-volatile silicone oils, they may preferably be chosen from the silicone oils of formula (I').

As regards the non-volatile phenylated silicone oils comprising at least one dimethicone fragment, they may be chosen from the compounds of formula (I) with radicals R such that the silicone is phenylated and comprises at least one dimethicone fragment; (II) with radicals R such that the silicone is phenylated and comprises at least one dimethicone fragment; (IV); (V) with non-zero p, in particular (VI) with non-zero p and especially the variants (A) and (B); (VII) with non-zero p, (IX) with radicals R such that the silicone is phenylated and comprises at least one dimethicone fragment, or mixtures thereof.

More particularly, the second oil(s) are chosen from non-volatile ester oils i), fatty alcohols ii) and mixtures thereof.

According to a third embodiment of the invention, the composition comprises a first oily phase containing at least a first oil chosen from non-volatile silicone oils and a second oily phase containing at least a second oil chosen from non-volatile hydrocarbon-based oils comprising at least two free hydroxyl groups and preferably at least three free hydroxyl groups.

More particularly, the said non-volatile hydrocarbon-based oils comprise at least one carboxylic ester group. Non-volatile hydrocarbon-based oils comprising three ester functions, of an acid comprising three monohydroxylated carboxylic functions and of a C₂-C₄ monoalcohol, are also suitable in this variant, as second oil.

According to a fourth preferred embodiment of the invention, the composition comprises a first oily phase containing at least a first oil chosen from phenylated non-volatile silicone oils not bearing a dimethicone fragment, and a second oily phase containing at least a second oil chosen from non-volatile hydrocarbon-based oils comprising at least two free hydroxyl groups and preferably at least three free hydroxyl groups, or from non-volatile apolar hydrocarbon-based oils, and also particularly at least a second oil chosen from non-volatile hydrocarbon-based oils comprising at least two free hydroxyl groups and preferably at least three free hydroxyl groups.

As regards the non-volatile phenylated silicone oils not bearing a dimethicone fragment, they are more particularly chosen from (I), with radicals R such that the silicone has no dimethicone fragment; (II) with radicals R such that the silicone has no dimethicone fragment, in particular formulae (III) and (III'); (V) with p = 0; (VI) with p=0; (VII) with p=0; (VIII); (IX) with radicals R such that the silicone has no dimethicone fragment; or mixtures thereof.

According to a particular embodiment of the invention, non-volatile apolar oil(s) are chosen from hydrogenated or non hydrogenated polydecenes, hydrogenated or non hydrogenated polybutenes and their mixtures.

Preferably, the non-volatile apolar hydrocarbon-based oils are chosen from oils with a viscosity of at least 50 cPs, preferably of at least 60 cPs.

In accordance with a fifth embodiment of the invention, the composition comprises a first oily phase containing at least a first oil chosen from non-volatile polar hydrocarbon-based oils and the second oily phase containing at least a second oil chosen from non-volatile or volatile, preferably non-volatile, apolar hydrocarbon-based oils.

Preferably, the first polar non-volatile hydrocarbon-based oil(s) are chosen from ester oils comprising at most one free hydroxyl group or not comprising any free hydroxyl group, and preferably also comprising at least three ester functions. The first polar non-volatile hydrocarbon-based oil(s) may also be chosen from oils comprising at least two free hydroxyl group; more particularly among oils also comprising at least one ester function or from polyhydrogenated alcohols and the mixtures of previously cited oils.

As for the second apolar non-volatile oil(s) are preferably chosen from hydrogenated or non hydrogenated polydecenes, hydrogenated or non hydrogenated polybutenes, hydrogenated or non hydrogenated polyisobutenes and their mixtures

In accordance with a sixth embodiment of the invention, the composition comprises a first oily phase comprising at least a first oil chosen from non-phenylated non-volatile silicone oils and a second oily phase containing at least a second oil chosen from phenylated non-volatile silicone oils optionally bearing a dimethicone fragment, or at least one silicone volatile oils.

In accordance with a final embodiment of the invention, the composition comprises a first oily phase comprising at least a first oil chosen from phenylated non-volatile silicone oils not bearing a dimethicone fragment, and a second oily phase containing at least a second oil chosen from phenylated non-volatile silicone oils bearing at least one dimethicone fragment, or at least one silicone volatile oils.

Preferably the first polar phenylated non-volatile siliconed oil(s) not bearing any dimethicone fragment are advantageously chosen from compounds of formula (II). The second non-volatile siliconed oil(s) bearing at least one dimethicone fragment are advantageously chosen from oils of formula (VI) particularly B) and for example silicones of formula (VII°)

As indicated previously, the composition may comprise at least one volatile oil. More particularly, the first oily phase and/or the second oily phase may comprise at least one volatile oil. It should be noted that the second oily phase may comprise only volatile oils. Reference may be made to that which has been detailed previously regarding the nature of these oils.

In addition, this or these volatile oils especially represent from 0.1% to 30% by weight, relative to the weight of the emulsion.

### Processes

The present invention is also directed towards processes for preparing the emulsion according to the invention.

The first oily phase is prepared in the first stage, and the second oily phase is prepared in a second stage.

Next, the process for preparing the emulsion may be continued, for example, according to the variants described below.

According to a first variant, the process for preparing the emulsion comprises the following steps, in this order:
- mixing of the first oily phase and of the second oily phase,
- emulsification of the mixture,
- introduction of the solid microparticles into the emulsion.

The first oily phase and the second oily phase are mixed together. The emulsification of these two oily phases leads to the creation of an interface, and more particularly a dispersion of one of the oily phases in the other oily phase.

In a second stage, the solid microparticles are added to the emulsion formed and the mixture is then agitated, either by means of a combination of shear forces or by ultrasonication.

According to a second variant, the process for preparing the emulsion is such that the solid microparticles are introduced into the first oily phase or into the second oily phase.

In this case, the process comprises the following steps, in this order:
- introduction of the solid microparticles into the first oily phase or into the second oily phase, respectively,
- introduction of the second oily phase or of the first oily phase, respectively,
- emulsification of the mixture.

According to this second variant, the microparticles are first introduced into one of the two oily phases, and a combination of shear forces or ultrasonication is then applied, so as to obtain a homogeneous dispersion of the said microparticles in the said oily phase. The other oily phase is then added.

According to a third variant, the process for preparing the emulsion comprises the following steps, in this order:
- simultaneous introduction of the solid microparticles, the first oily phase and the second oily phase,
- emulsification of the mixture.

According to this variant, the emulsion is obtained by mixing the solid microparticles and the two oily phases with vigorous agitation.

Emulsification takes place by subjecting the mixture of the two oily phases and the solid microparticles to a combination of shear forces or ultrasonication, to obtain homogeneity thereof.

The term "homogeneity" of an emulsion is intended to denote an emulsion in which the drops of inner phases are uniformly dispersed in the continuous or outer oily phase.

The drops of dispersed phases in the emulsion may be very fine, in particular ranging from 0.1 to 10 µm, or may be coarser, in particular ranging from 10 µm to 1 cm.

A person skilled in the art may choose the conditions and the device that are the best suited for obtaining the combination of forces necessary for obtaining the targeted type of emulsion, especially for obtaining the targeted droplet size.

This combination of forces may be obtained by subjecting the first and second oily phases or the emulsion to manual shaking or to mechanical stirring with a blender such as a Moritz, Rayneri or Ultra-Turrax blender, or alternatively by ultrasonic homogenization.

The speed of blending or stirring for obtaining a homogeneous phase or emulsion may depend on various factors such as its composition or its volume.

The various stirring parameters, especially the speed, may be determined by a person skilled in the art on the basis of his general knowledge and, where appropriate, by means of a few routine tests.

Usually, this operation is performed at a temperature between 10 and 50°C; advantageously at a temperature between 15 and 30°C.

In the description and in the examples that follow, unless otherwise mentioned, the percentages are weight percentages and the ranges of values written in the form "between ... and ..." include the stated lower and upper limits.

The examples below are presented as non-limiting illustrations of the field of the invention.

### EXAMPLES

### ULTRASONICATION EXAMPLE

### Series 1: Emulsions prepared using particles of silicone resin bowl type - diameter: 2.3 µm ± 0.9

Sonicator XL machine from Misonix Incorporated for 1 minute to 1 minute 5 seconds with a power of 110 W to 165 W; 25°C.

| **Starting materials** | **Example 1 (Invention)** | **Example 2 (Invention)** | **Example 3 (Invention)** | **Example 4 (Invention)** |
|---|---|---|---|---|
| Castor oil (g/100 g) | 47.5 | 46.25 | 55.5 | 64.75 |
| PDMS oil 100 cSt (g/100 g) | 47.5 | 46.25 | 37 | 27.75 |
| Silicone resin bowl microparticles (g/100 g) NLK-506 from Takemoto Oil & Fat | 5 | 7.5 | 7.5 | 7.5 |
| Total | 100 | 100 | 100 | 100 |
| Ultrasonication power (watts)/time (seconds) | 165 W/(35 s + 30 s) | 137.5 W/(35 s + 30 s) | 137.5 W/(35 s + 30 s) | 137.5 W/(35 s + 30 s) |
| Result | Pickering emulsion | Pickering emulsion | Pickering emulsion | Pickering emulsion |

| **Starting materials** | **Example 5 (Invention)** |
|---|---|
| Castor oil (g/100 g) | 47.5 |
| Hydrogenated pol yisobutene (Parleam ® from NOF(g/100 g) | 47.5 |
| Silicone resin bowl microparticles (g/100 g) NLK-506 from Takemoto Oil & Fat | 5 |
| Total | 100 |
| Ultrasonication power (watts)/time (seconds) | 137.5 W/(35 s + 30 s) |
| Result | Pickering emulsion |

### Preparation protocol:

The emulsions are prepared by ultrasonication in two steps:
- "initiation" phase for 35 s to create interfaces,
- then "end of step" phase with addition of the particles for 30 s.

The power applied is that specified in the above table for each emulsion.

### Series 2: Emulsions prepared using particles of PMMA bowl type - diameter: 11.8 µm ± 1.5

| **Starting materials** | **Example 1 (Invention)** | **Example 2 (Invention)** |
|---|---|---|
| Castor oil (g/100 g) | 46.25 | 47.5 |
| PDMS oil 100 cSt (90×10⁻³Pa.s) (g/100 g) | 46.25 | 47.5 |
| PMMA bowl microparticles (g/100 g) Sepimat H 10 from Matsumoto Yushi-Seiyaku | 7.5 | 5 |
| Total | 100 | 100 |
| Ultrasonication power (watts)/time (seconds) | 137.5 W/(35 s + 30 s) | 165 W/(35 s + 30 s) |
| Results | Pickering emulsion | Pickering emulsion |

| **Starting materials** | **Example 3 (Invention)** | **Example 4 (Invention)** | **Example 5 (Invention)** |
|---|---|---|---|
| Castor oil (g/100 g) | 47.5 | - | - |
| Triethyle citrate (g/100 g) | - | 47.5 | 42,75 |
| PENTYLENE GLYCOL | - | - | 4,75 |
| PDMS oil 100 cSt (g/100 g) | - | - | 47.5 |
| Hydrogenated pol yisobutene (Parleam ® from NOF(g/100 g) | 47.5 | 47.5 | - |
| PMMA bowl microparticles (g/100 g) Sepimat H 10 from Matsumoto Yushi-Seiyaku | 5 | 5 | 5 |
| Total | 100 | 100 | 100 |
| Ultrasonication power (watts)/time (seconds) | 137.5 W/(35 s + 30 s) | 137.5 W/(35 s + 30 s) | 137.5 W/(35 s + 30 s) |
| Result | Pickering emulsion | Pickering emulsion | Pickering emulsion |

### Preparation protocol:

The emulsions are prepared by ultrasonication in two steps, at 25°C:
- "initiation" phase for 35 s to create interfaces,
- then "end of step" phase with addition of the particles for 30 s.

The power applied is that specified in the above table for each emulsion.

### EXAMPLE OF FORMULATION USING AN ULTRA-TURRAX BLENDER

| **Starting materials** | | **Composition (%) (Invention)** |
|---|---|---|
| **Oil 1** | Castor oil | 47.5 |
| **Oil 2** | PDMS | 47.5 |
| **Stabilizing particles** | Silicone resin bowl microparticles NLK-506 from Takemoto Oil & Fat | 5 |
| | Total | 100 |

In a first stage, the stabilizing particles are introduced with stirring using a Rayneri blender (speed adjusted so as to have a strong vortex: vortex height equal to 3/4 of the height of the mixture) into oil 2.

Oil 1 placed beforehand in a beaker is stirred for 5 minutes using an IKA T25 Ultra-Turrax blender (20 000 rpm).

The mixture of oil 2 with the particles is then introduced into oil 1 with continued stirring. The whole is stirred for 5 minutes.

The process is performed at 25°C.

A Pickering emulsion is obtained.

### EXAMPLE OF FORMULATION USING A RAYNERI BLENDER

| **Starting materials** | | **Composition (%) (Invention)** |
|---|---|---|
| **Oil 1** | Castor oil | 47.5 |
| **Oil 2** | PDMS | 47.5 |
| **Stabilizing particles** | Silicone resin bowl microparticles NLK-506 from Takemoto Oil & Fat | 5 |
| | Total | 100 |

The silicone oil, then the hydrocarbon-based oil and then the fillers are placed in a beaker, at 25°C.

This mixture is then blended for 15 minutes using a Rayneri blender at 1000 rpm.

A Pickering emulsion is obtained.

### Series 3: Emulsions prepared using particles of silicone resin rugby balls

Sonicator XL machine from Misonix Incorporated for 1 minute to 1 minute 5 seconds with a power of 110 W to 165 W; 25°C.

| **Starting materials** | **Example 1 (Out of invention)** | **Example 2 (Out of invention)** | **Example 3 (Out of invention)** |
|---|---|---|---|
| Triethyle citrate (g/100 g) | 47.5 | - | - |
| Castor oil (g/100 g) | - | 47.5 | 47.5 |
| PDMS oil 100 cSt (g/100 g) | 47.5 | - | - |
| Hydrogenated pol yisobutene (Parleam ® from NOF(g/100 g) | - | 47.5 | - |
| isododecane | - | - | 47.5 |
| silicone resin rugby balls particles NLK-602 of Takemoto Oil & Fat (*) | 5 | 5 | 5 |
| Total | 100 | 100 | 100 |
| Ultrasonication power (watts)/time (seconds) | 137.5 W/(35 s + 30 s) | 137.5 W/(35 s + 30 s) | 137.5 W/(35 s + 30 s) |
| Result | Pickering emulsion | Pickering emulsion | Pickering emulsion |

| | | | |
|---|---|---|---|
| (*) these particles are preared acording to the teaching of EP2433979 | | | |

| **Starting materials** | **Example 4 (Out of invention)** |
|---|---|
| MIRASIL DM 350 Bluestar (g/100 g) | 47.5 |
| Trimethyl pentaphenyl trisiloxane(Dow Corning PH-1555 HRI Cosmetic Fluid) (g/100 g) | 47.5 |
| silicone resin rugby balls particles (NLK-602 of Takemoto Fat & Oil) (*) | 5 |
| Total | 100 |
| Ultrasonication power (watts)/time (seconds) | 137.5 W/(35 s + 30 s) |
| Result | Pickering emulsion |

| | |
|---|---|
| (*) these particles are preared acording to the teaching of EP2433979 | |

### Preparation protocol:

The emulsions are prepared by ultrasonication in two steps:
- "initiation" phase for 35 s to create interfaces,
- then "end of step" phase with addition of the particles for 30 s.

The power applied is that specified in the above table for each emulsion.

## Claims

1. Oil/oil emulsion stabilized with solid particles comprising at least:
- a first oily phase comprising at least a first non-volatile oil chosen from silicone oils, hydrocarbon-based oils and fluoro oils,
- a second oily phase comprising at least a second non-volatile or volatile oil, which is immiscible with the first oil(s), at 25°C,
- solid microparticles having at least one curved part and at least one breakage of curvature of the said curved part, in which the solid microparticles comprise at least one concave part and at least one convex part.

2. Oil/oil emulsion according to Claim 1, in which the microparticles have a form chosen from forms of "bowl", "golfball" and "polytope" type.

3. Oil/oil emulsion according to any one of the preceding claims, in which the microparticles are such that their largest dimension ranges from 0.1 to 100 µm, preferably from 0.1 to 50 µm and more preferably from 0.5 to 20 µm.

4. Oil/oil emulsion according to any one of the preceding claims, in which the total amount of solid microparticles comprising at least one curved part and at least one breakage of curvature of the said curved part ranges from 1% to 10% and preferably from 2% to 7% by weight relative to the total weight of the emulsion.

5. Oil/oil emulsion according to any one of the preceding claims, in which the first oily phase contains at least a first oil chosen from non-phenylated non-volatile silicone oils or phenylated non-volatile silicone oils bearing at least one dimethicone fragment, and the second oily phase contains at least a second oil chosen from non-volatile hydrocarbon-based oils comprising not more than one free hydroxyl group or not comprising any.

6. Oil/oil emulsion according to any one of Claims 1 to 4, in which the first oily phase contains at least a first oil chosen from phenylated non-volatile silicone oils not bearing a dimethicone fragment, and the second oily phase contains at least a second oil chosen from non-volatile hydrocarbon-based oils comprising at least two free hydroxyl groups and preferably at least three free hydroxyl groups, or from non-volatile apolar hydrocarbon-based oils.

7. Oil/oil emulsion according to any one of Claims 1 to 4, in which the first oily phase contains at least a first oil chosen from non-volatile polar hydrocarbon-based oils and the second oily phase contains at least a second oil chosen from non-volatile apolar hydrocarbon-based oils.

8. Oil/oil emulsion according to any one of Claims 1 to 4, in which the first oily phase contains at least a first oil chosen from non-phenylated non-volatile silicone oils and phenylated non-volatile silicone oils not bearing a dimethicone fragment, and the second oily phase contains at least a second oil chosen from phenylated non-volatile silicone oils bearing at least one dimethicone fragment, or at least one silicone volatile oils.

9. Oil/oil emulsion according to any one of the preceding claims, in which the oil content of the first oily phase represents from 5% to 95% by weight and preferably from 30% to 70% by weight, relative to the weight of the emulsion and the oil content of the second oily phase represents from 5% to 95% by weight and preferably from 30% to 70% by weight, relative to the weight of the emulsion.

10. Cosmetic composition comprising, in a physiologically acceptable medium, at least one oil/oil emulsion according to any one of Claims 1 to 9.

11. Use of solid microparticles having at least one curved part and at least one breakage of curvature of the said curved part, in which the solid microparticles comprise at least one concave part and at least one convex part, for stabilizing an oil/oil emulsion comprising at least a first oily phase comprising at least a first non-volatile oil chosen from silicone oils, hydrocarbon-based oils and fluoro oils, and at least a second oily phase comprising at least a second oil that is immiscible with the first oil(s) at 25°C.

## Patentansprüche

1. Öl/Öl-Emulsion stabilisiert mit Feststoffteilchen, die mindestens umfasst:
- eine erste ölige Phase, die mindestens ein erstes nichtflüchtiges Öl umfasst, das aus Silikonölen, kohlenwasserstoffbasierten Ölen und Fluorölen ausgewählt ist,
- eine zweite ölige Phase, die mindestens ein zweites nichtflüchtiges oder flüchtiges Öl umfasst, das mit dem bzw. den ersten Ölen bei 25 °C unvermischbar ist,
- Feststoffmikroteilchen mit mindestens einem gekrümmten Teil und mindestens einer Krümmungsbrechung des besagten gekrümmten Teils, wobei die Feststoffmikroteilchen mindestens einen konkaven Teil und mindestens einen konvexen Teil umfassen.

2. Öl/Öl-Emulsion nach Anspruch 1, wobei die Mikroteilchen eine Form aufweisen, die ausgewählt ist aus Formen des Typs "Schale", "Golfball" und "Polytop".

3. Öl/Öl-Emulsion nach einem der vorstehenden Ansprüche, wobei die Mikroteilchen derart sind, dass ihre größte Abmessung von 0,1 bis 100 µm, bevorzugt von 0,1 bis 50 µm und mehr bevorzugt von 0,5 bis 20 µm reicht.

4. Öl/Öl-Emulsion nach einem der vorstehenden Ansprüche, wobei die Gesamtmenge an Feststoffmikroteilchen mindestens einen gekrümmten Teil und mindestens eine Krümmungsbrechung des besagten gekrümmten Teils von 1 Gew.-% bis 10 Gew.-% und bevorzugt von 2 Gew.-% bis 7 Gew.-% relativ zum Gesamtgewicht der Emulsion umfasst.

5. Öl/Öl-Emulsion nach einem der vorstehenden Ansprüche, wobei die erste ölige Phase mindestens ein erstes Öl enthält, das aus nichtphenylierten nichtflüchtigen Silikonölen oder phenylierten nichtflüchtigen Silikonölen ausgewählt ist, die mindestens ein Dimethiconfragment enthalten, und die zweite ölige Phase mindestens ein zweites Öl enthält, das aus nichtflüchtigen kohlenwasserstoffbasierten Ölen, die nicht mehr als eine freie Hydroxylgruppe oder überhaupt keine umfassen, ausgewählt ist.

6. Öl/Öl-Emulsion nach einem der Ansprüche 1 bis 4, wobei die erste ölige Phase mindestens ein erstes Öl enthält, das aus phenylierten nichtflüchtigen Silikonölen, die kein Dimethiconfragment enthalten, ausgewählt ist, und die zweite ölige Phase mindestens ein zweites Öl enthält, das aus nichtflüchtigen kohlenwasserstoffbasierten Ölen, die mindestens zwei freie Hydroxylgruppen und bevorzugt mindestens drei freie Hydroxylgruppen umfassen, oder aus nichtflüchtigen apolaren kohlenwasserstoffbasierten Ölen ausgewählt ist.

7. Öl/Öl-Emulsion nach einem der Ansprüche 1 bis 4, wobei die erste ölige Phase mindestens ein erstes Öl enthält, das aus nichtflüchtigen polaren kohlenwasserstoffbasierten Ölen ausgewählt ist, und die zweite ölige Phase mindestens ein zweites Öl enthält, das aus nichtflüchtigen apolaren kohlenwasserstoffbasierten Ölen ausgewählt ist.

8. Öl/Öl-Emulsion nach einem der Ansprüche 1 bis 4, wobei die erste ölige Phase mindestens ein erstes Öl enthält, das aus nichtphenylierten nichtflüchtigen Silikonölen und phenylierten nichtflüchtigen Silikonölen ausgewählt ist, die kein Dimethiconfragment enthalten, und die zweite ölige Phase mindestens ein zweites Öl enthält, das aus phenylierten nichtflüchtigen Silikonölen, die mindestens ein Dimethiconfragment enthalten, oder mindestens einem flüchtigen Silikonöl ausgewählt ist.

9. Öl/Öl-Emulsion nach einem der vorstehenden Ansprüche, wobei der Ölanteil der ersten öligen Phase 5 Gew.-% bis 95 Gew.-% und bevorzugt 30 Gew.-% bis 70 Gew.-%, relativ zum Gewicht der Emulsion repräsentiert und der Ölanteil der zweiten öligen Phase 5 Gew.-% bis 95 Gew.-% und bevorzugt 30 % bis 70 Gew.-%, relativ zum Gewicht der Emulsion repräsentiert.

10. Kosmetikzusammensetzung, die, in einem physiologisch verträglichen Medium, mindestens eine Öl/Öl-Emulsion nach einem der Ansprüche 1 bis 9 umfasst.

11. Gebrauch von Feststoffmikroteilchen mit mindestens einem gekrümmtem Teil und mindestens einer Krümmungsbrechung des besagten gekrümmten Teils, wobei die Feststoffmikroteilchen mindestens einen konkaven Teil und mindestens einen konvexen Teil umfassen, zum Stabilisieren einer Öl/Öl-Emulsion, die mindestens eine erste ölige Phase, die mindestens ein erstes nichtflüchtiges Öl umfasst, das aus Silikonölen, kohlenwasserstoffbasierten Ölen und Fluorölen ausgewählt ist, und mindestens eine zweite ölige Phase umfasst, die mindestens ein zweites Öl umfasst, das mit dem bzw. den ersten Ölen bei 25 °C unvermischbar ist.

## Revendications

1. Emulsion Huile / Huile stabilisée avec des particules solides, comprenant au moins :
- une première phase huileuse comprenant au moins une première huile non volatile choisie parmi les huiles siliconées, les huiles hydrocarbonées et les huiles fluorées,
- une deuxième phase huileuse comprenant au moins une deuxième huile non volatile ou volatile, non miscible avec la ou les premières huiles à 25°C,
- des microparticules solides présentant au moins une partie courbe et au moins une rupture de courbure de ladite partie courbe, lesdites microparticules solides comprenant au moins une partie concave et au moins une partie convexe.

2. Emulsion Huile / Huile selon la revendication 1, dans laquelle les microparticules présentent une forme choisie parmi les formes de type « bols », « balles de golf » et « polytopes ».

3. Emulsion Huile / Huile selon l'une quelconque des revendications précédentes, dans laquelle les microparticules sont telles que leur plus grande dimension va de 0,1 à 100 µm, de préférence de 0,1 à 50 µm, et de manière encore préférée de 0,5 à 20 µm.

4. Emulsion Huile / Huile selon l'une quelconque des revendications précédentes, dans laquelle la quantité totale de microparticules solides comprenant au moins une partie courbe et au moins une rupture de la courbure de ladite partie courbe, va de 1 à 10 %, de préférence de 2 à 7 % en poids, par rapport au poids total de l'émulsion.

5. Emulsion Huile / Huile selon l'une quelconque des revendications précédentes, dans laquelle la première phase huileuse contient au moins une première huile choisie parmi les huiles siliconées non volatiles non phénylées et les huiles siliconées non volatiles phénylées portant au moins un fragment diméthicone, et la deuxième phase huileuse contient au moins une deuxième huile choisie parmi les huiles hydrocarbonées non volatiles comprenant au plus un groupement hydroxyle libre ou n'en comprenant aucun.

6. Emulsion Huile / Huile selon l'une quelconque des revendications 1 à 4, dans laquelle la première phase huileuse contient une première huile choisie parmi les huiles siliconées non volatiles phénylées ne portant pas de fragment diméthicone, et la deuxième phase huileuse contient au moins une deuxième huile choisie parmi les huiles hydrocarbonées non volatiles comprenant au moins deux groupements hydroxyle libres et de préférence au moins trois groupements hydroxyles libres, ou parmi les huiles hydrocarbonées apolaires non volatiles.

7. Emulsion Huile / Huile selon l'une quelconque des revendications 1 à 4, dans laquelle la première phase huileuse contient au moins une première huile choisie parmi les huiles hydrocarbonées polaires non volatiles et la deuxième phase huileuse contient au moins une deuxième huile choisie parmi les huiles hydrocarbonées apolaires non volatiles.

8. Emulsion Huile / Huile selon l'une quelconque des revendications 1 à 4, dans laquelle la première phase huileuse contient au moins une première huile choisie parmi les huiles siliconées non volatiles non phénylées et les huiles siliconées non volatiles phénylées ne portant pas de fragment diméthicone, et la deuxième phase huileuse contient au moins une deuxième huile choisie parmi les huiles siliconées non volatiles phénylées portant au moins un fragment diméthicone, ou au moins une huile volatile siliconée.

9. Emulsion Huile / Huile selon l'une quelconque des revendications précédentes, dans laquelle la teneur en huile de la première phase huileuse représente de 5 à 95 % en poids et de préférence de 30 à 70 % en poids par rapport au poids de l'émulsion, et la teneur en huile de la deuxième phase huileuse représente de 5 à 95 % en poids et de préférence de 30 à 70 % en poids par rapport au poids de l'émulsion.

10. Composition cosmétique comprenant, dans un milieu physiologiquement acceptable, au moins une émulsion Huile / Huile selon l'une quelconque des revendications 1 à 9.

11. Utilisation de microparticules solides ayant au moins une partie courbe et au moins une rupture de courbure de ladite partie courbe, lesdites microparticules solides comprenant au moins une partie concave et au moins une partie convexe, pour stabiliser une émulsion Huile / Huile comprenant au moins une première phase huileuse comprenant au moins une première huile non volatile choisie parmi les huiles siliconées, les huiles hydrocarbonées et les huiles fluorées, et au moins une deuxième phase huileuse comprenant au moins une deuxième huile non miscible avec la ou les premières huiles à 25°C.
